# EUROPEAN PATENT APPLICATION

(11) **EP 3 593 759 A1**
(43) Date of publication of application: **15.01.2020**
(21) Application number: 18182805.4
(22) Date of filing: 10.07.2018
(51) Int. Cl.: A61F 2/24, A61B 17/04

(54) **A CHAIN ANNULOPLASTY RING AND DELIVERY SYSTEM**

(71) Applicant: Syntach AG, 8200 Schaffhausen (CH)
(72) Inventor: Solem, Jan Otto, 237 42 Bjärred (SE); Solem, Kristian, 231 45 Trelleborg (SE); Engvall, Daniel, 302 93 Halmstad (SE); Krüger, Victoria, 238 43 Oxie (SE); Wolff, Martin, 223 55 Lund (SE); Berg, Jonathan, 244 60 Furulund (SE); Spånberg, André, 227 30 Lund (SE)
(74) Representative: KIPA AB

(57) **Abstract**

An at least partial annuloplasty ring for transcatheter cardiac valve treatment is disclosed. The annuloplasty ring has a plurality of separate chain segments serially interlinked and articulated to a chain. The chain has a substantially elongate delivery configuration and a curved deployment configuration. At least one of said chain segments has at least one tissue anchor attached to it, wherein the tissue anchor is movably arranged at least partly within the chain segment in the delivery configuration and protrudes from the chain segment in the deployment configuration in order to anchor with cardiac tissue.

## Description

### BACKGROUND OF THE INVENTION

### Field of the Invention

This invention pertains in general to the field of medical implantable devices for improving function of a cardiac valve. More particularly, examples of the invention relate to annuloplasty rings for treatment of cardiac valve insufficiencies.

### Description of Prior Art

This section is intended to introduce the reader to various aspects of art that may be related to various aspects of the present disclosure, which are described and/or claimed below. This discussion is believed to be helpful in providing the reader with background information to facilitate a better understanding of the various aspects of the present disclosure. Accordingly, it should be understood that these statements are to be read in this light, and not as admissions of prior art.

Heart valve defects, such as regurgitation, may be caused by a relaxation of the tissue surrounding a heart valve (e.g. the mitral valve or tricuspid valve). This causes the valve opening to enlarge, which prevents the valve from sealing properly. Such heart conditions are commonly treated by a procedure during which an annuloplasty ring is fixed or secured around the valve. Cinching or securing the tissue to the ring can restore the valve opening to its approximate original size and operating efficiency.

Typically, annuloplasty rings have been implanted during open heart surgery, so the annuloplasty ring can be sewn into the valve annulus. Open heart surgery is a highly invasive procedure with a high risk for the patient in many clinical aspects. Minimally invasive concepts for delivery of annuloplasty implants are therefore under development. However, most commonly, a reshaping of the annulus is performed, e.g. by introducing a reshaping device in the coronary sinus surrounding the mitral valve annulus. The shape is then fixated by an annuloplasty ring being affixed to the annulus tissue. Several concepts are pursued, but all suffer from drawbacks.

Thus, there is a need for a new improved transcatheter delivered annuloplasty ring allowing for flexibility of the procedure.

### Summary of the Invention

Accordingly, embodiments of the present invention preferably seek to mitigate, alleviate or eliminate one or more deficiencies, disadvantages or issues in the art, such as the above-identified, singly or in any combination by providing devices, systems, and methods according to the appended independent patent claims. Further embodiments of the invention are defined in the dependent claims, wherein features for the second and subsequent aspects of the invention are as for the first aspect mutatis mutandis. The disclosure may include further inventions not presently claimed.

In an aspect of the disclosure, an implantable medical device for cardiac valve treatment is provided, which includes an at least partial annuloplasty ring. The annuloplasty ring has a plurality of separate chain segments which are serially interlinked and articulated thus forming a chain. The chain has a substantially elongated delivery configuration and a curved deployment configuration. A minimum of one chain segment of the chain has at least one tissue anchor movably attached to it. The tissue anchor is arranged in a movable state at least partially within the chain segment in the delivery configuration. The tissue anchor of at least one of the chain segments is preferably arranged to protrude from the chain segment in the deployment configuration oriented towards adjacent cardiac tissue in order to anchor with the cardiac tissue.

An at least partial annuloplasty ring for transcatheter cardiac valve treatment is thus provided. The term transcatheter means that an annuloplasty ring and related devices, if any, are delivered through a catheter from outside of the patient's body to a cardiac tissue site within the patient via a catheter, i.e. a tubular elongated device to accommodate the annuloplasty ring and related devices (sequentially if so needed) in an inner lumen thereof during delivery. The catheter distal end is brought to the site while the proximal end is kept outside of the patient. The device(s) are then moved forward to the lumen until deployment out of the catheter's distal end out of the catheter to the cardiac tissue site. Transcatheter delivery includes for instance intercostal access, transvascular access, transapical access, etc. A specific example is provided in the detailed description herein.

In an aspect of the disclosure, a deployment apparatus for a chain annuloplasty implant is provided. The apparatus includes a double lumen catheter, and a wire with a screw driver unit.

In an aspect of the disclosure, a method of deployment of a chain annuloplasty implant is provided. The method includes providing a chain implant 100. The method includes delivering a chain implant to an annulus of cardiac valve, anchoring of the chain implant 100 to the annulus by screwing screw tissue anchors 120 into the annulus. In case, the chain implant is not a pure anchor implant, but an annuloplasty implant, the method includes optionally foreshortening the chain implant 100 for the annuloplasty. The method further optionally includes stabilizing of the chain implant by locking one or more arms 141, 142, 143.

In an aspect of the disclosure, a method of treating a cardiac valve is provided. The method includes performing an annuloplasty by foreshortening an anchored chain ring implant and preferably patient specific adaptation of a curvature of said ring implant; stabilizing said chain ring implant with one or more arms 141, 142, 143; and optionally connecting to said arms a cardiac valve tissue anchor, a cardiac assist device, or a valve repair or replacement unit.

### BRIEF DESCRIPTION OF THE DRAWINGS

Fig. 1 is a schematic illustration of a heart and its anatomical structures in a partly cross-sectional schematic illustration of a human heart depicting structures that are involved.
Fig. 2 is a schematic illustration of a heart and its related cardiac valves as well as the cardiac axis.
Fig. 3 is a top view of an annuloplasty ring chain with tissue anchoring elements.
Fig. 4 is a 3D view of the ring of Fig. 3 with protruding tissue anchors.
Fig. 5 is a 3D view of the ring of Fig. 3 with stabilizing arms attached to it.
Fig. 6 is a detailed view of the ring of Fig. 5 illustrating tissue anchor deployment and tissue anchors arranged within chain segments in a state upon deployment from a delivery catheter.

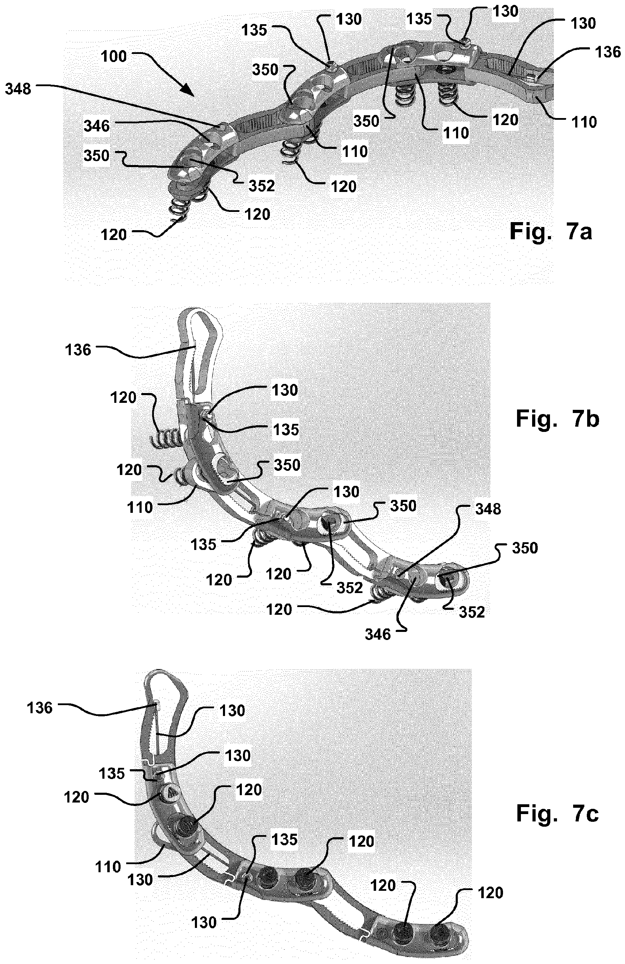

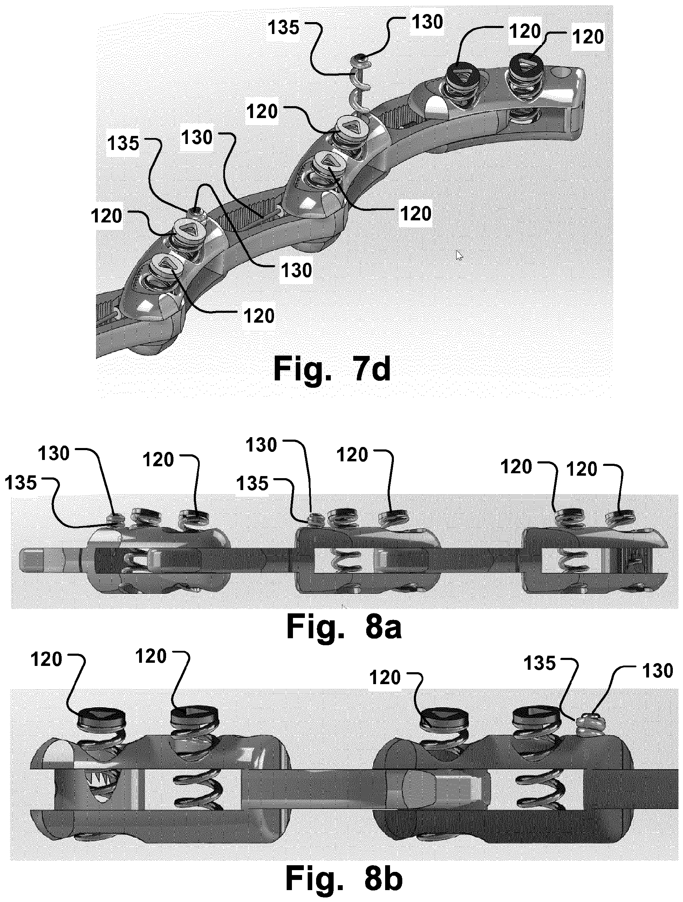

Figs. 7a,7b,7c,7d are top elevated 3D views of a part of an elongated annuloplasty chain with extended adjacent chain elements not restricted by a catheter and in the transition to a ring shape as well as with protruding tissue anchors for illustration purposes.
Figs. 8a, 8b are detailed front 3D views of a chain ring.

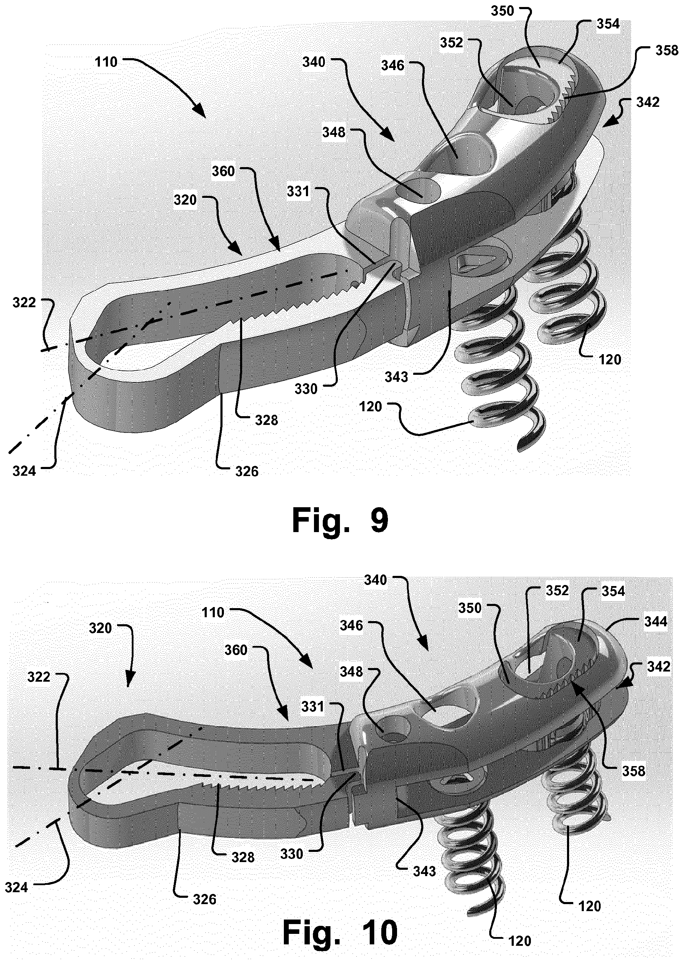

Figs. 9 and 10 are different top elevated 3D view of a part of an elongated annuloplasty chain with a chain element and two tissue anchors protruding from the element.
Fig. 11 is a top 3D view of the chain element corresponding to Figs. 9-10.
Fig. 12 is a bottom lateral 3D view of the chain element corresponding to Figs. 9-11.
Fig. 13 is a bottom lateral 3D view of two chain elements during foreshortening.
Fig. 14 is an elevated top 3D view of two chain elements after foreshortening.
Fig. 15 is an elevated top 3D view of chain ring with anchors after foreshortening annuloplasty and with three stabilizing arms locking in a center of the chain ring.
Fig. 16 is a front 3D view of the chain ring of Fig. 15.
Fig. 17 is an elevated top 3D view of chain ring illustrating the locking unit including the locked arms.
Figs. 18, 19 and 20 are a top view and an elevated 3D view of a chain ring in the elongate delivery configuration.
Fig. 21 is a schematic illustration of the annuloplasty ring in an inside view of the lumen.
Fig. 22 is illustrating a delivery view of a chain annuloplasty ring with an end chain segment of a chain protruding from a delivery catheter.
Figs. 23a-c are schematic illustrations of percutaneous transcatheter access paths to the heart.
Fig. 24a-b are schematic illustrations that show a direct access path to a cardiac valve via an incision in the skin and a puncture 95 in the apex of the heart.
Figs. 25-26 are schematic illustrations that depict an example for complete annuloplasty ring implantation of the medical device.
Fig.27 is a schematic illustration of a multi lumen catheter 150 arranged in a delivery catheter 155 and an introducer catheter 123.
Figs. 28 and 29 are cross-sectional illustrations of two lumina in the multi lumen catheter 150 from Fig. 27.
Figs. 30-42 are schematic step illustrations of a method for deployment of a chain annuloplasty implant.
Figs. 43a-b, 44a-b, 45a-b, and 46 are schematic illustrations of separate chain segments and its components during various assembly stages.
Fig. 47a-c are schematic illustrations of different chain annuloplasty ring configurations.

### DESCRIPTION OF THE PREFERRED EMBODIMENTS

Specific embodiments of the invention will now be described with reference to the accompanying drawings. This invention may, however, be embodied in many different forms and should not be construed as limited to the embodiments set forth herein; rather, these embodiments are provided so that this disclosure will be thorough and complete, and will fully convey the scope of the invention to those skilled in the art. The terminology used in the detailed description of the embodiments illustrated in the accompanying drawings is not intended to be limiting of the invention. In the drawings, like numbers refer to like elements.

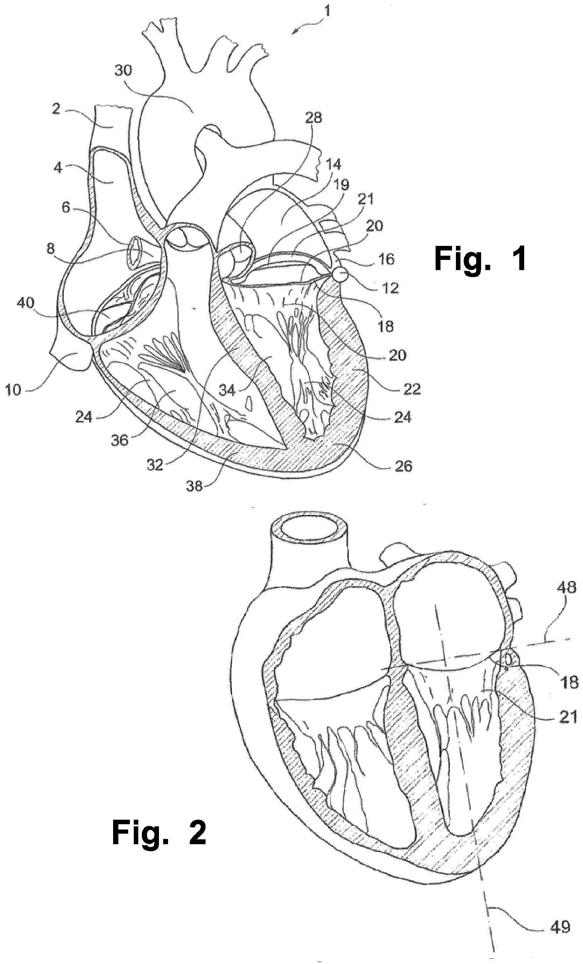

Fig. 1 depicts the anatomical structures of the heart 1, of which at least some are involved in embodiments of the invention, 2 is the Superior Vena Cava (SVC), 4 is the right atrium (RA), 6 is the Coronary Sinus (CS) ostium, 8 is the CS first part, 10 is the Inferior Vena Cava (IVC), 12 is the Great Cardiac Vein (GCV) at the level of the Mitral Valve (MV) annulus 18, 14 is the Left Atrium cavity (LA), 16 is the LA wall, 19 is the whole mitral valve, 20 is the anterior leaflet and 21 is the posterior leaflet of the mitral valve, 22 is the Left Ventricle (LV) muscular wall, 24 are the papillary muscles connected to the chordae, 26 is the apex of the left ventricle, 28 is the aortic valve, 30 the aorta ascendens, 32 the inter-ventricular muscular septum, 34 the left ventricular cavity and 36 the right ventricular cavity, 38 is the right ventricular muscular wall and 40 is the tricuspid valve.

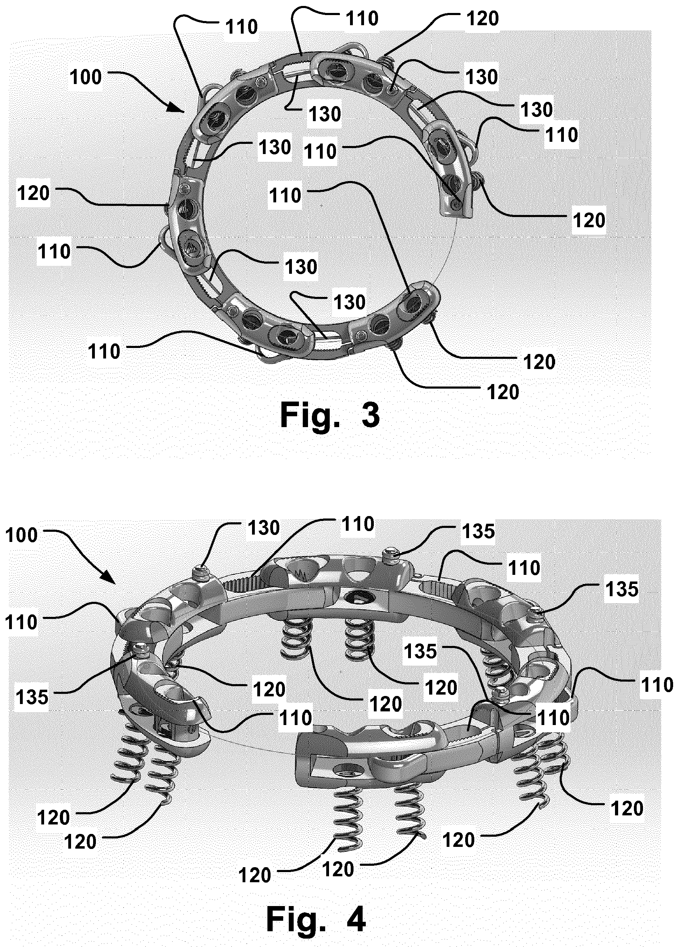

Fig. 2 shows the cardiac valve plane 48 in relation to the cardiac axis 49 of the left ventricle. In an example an implantable medical device for cardiac valve treatment is provided, which includes an at least partial annuloplasty ring 100. The annuloplasty ring 100 has a plurality of separate chain segments 110 which are serially interlinked and articulated to a chain 100. The chain 100 has a first substantially elongated delivery configuration and a second curved deployment configuration. At least one of the chain segments 110 includes at least one movably attached tissue anchor 120. Fig. 3 is a top view of an annuloplasty ring 100 chain with tissue anchoring elements 120. Fig. 4 is a 3D view of the ring 100 of Fig. 3 with protruding tissue anchors 120.

### Description of chain segment 110 elements of a chain ring 100:

A chain segment 110 may include one or more of the following items or features of a chain segment body. The body preferably has a curved shape 360 and includes a first end portion 320 to engage with a first adjacent chain segment's second end portion 340.

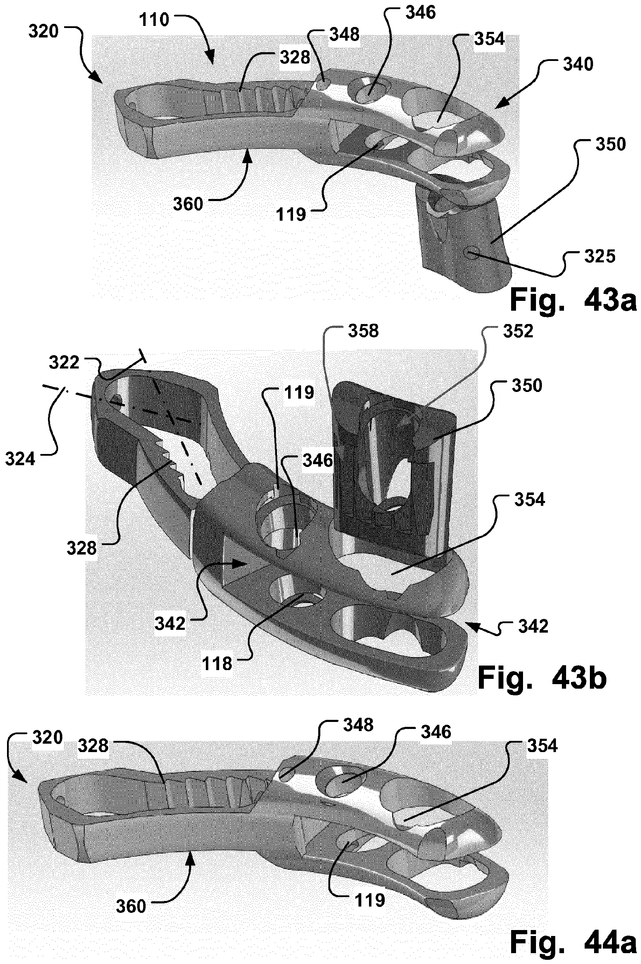

This first end portion 320 is matingly received therein. In the illustrated examples (see in particular the assembly described below in relation to Figs.43-46), the first end portion 320 is a protrusion configured to engage with a corresponding groove or slot 342 of portion 340. Similar matingly connecting interfaces may be provided in other examples. A stop surface 343 prevents further movement of the adjacent first end portion 320 when inserted into the slot 342. A second tissue anchor hole 346 is provided for receiving a tissue anchor 120. This tissue anchor, when inserted and blocking slot 342 functions as a stop to prevent unintended teeth 328/358 engagement and non-reversible foreshortening until anchored into tissue and freeing the slot 342.

A rounded end 344 is provided for tissue friendly properties and easy tissue overgrowth when implanted.

The second end portion 340 is arranged to engage with another adjacent chain segment in the opposite direction of the chain. In examples, this second end portion 340 has teeth 358 matingly receiving teeth 328 of the adjacent chain segments first portion. A gap 331 allows for the arm portion with teeth 328 to flex over teeth 358. A tow 330 limits the flexible movement and prevents the arm to slide out too far. The first end portion 320 has an interior groove with the teeth 328 arranged therein along a first axis 322 as well as a distal portion extending beyond the teeth and outwardly along a second axis 324 at an angle to the first axis 322 to allow for the substantially elongate delivery configuration (see below). The inner groove receives therein the attachment unit 350 of the adjacent chain segment upon assembly. Attachment unit is arranged to move relative the interior groove and along axis 322 as well as pivot around a knee 326 along axis 324.

Attachment unit 350 has matingly engaging teeth 358 arranged at its outside to provide a non-reversible engagement and relative movement of the two adjacent chain segments 110. Attachment unit 350 has an interior hole 352. In interior hole 352 elements can be received, like tissue anchor 120 or a (pivotable) attachment of an arm. Hence, attachment unit 350 has several advantageous functions, including pivotable holding together two chain segments, provide non-reversible foreshortening (teeth), and attachment of further elements. Attachment unit 350 is oval and received in an oval hole 354 to prevent rotation of the attachment unit 350 in the hole.

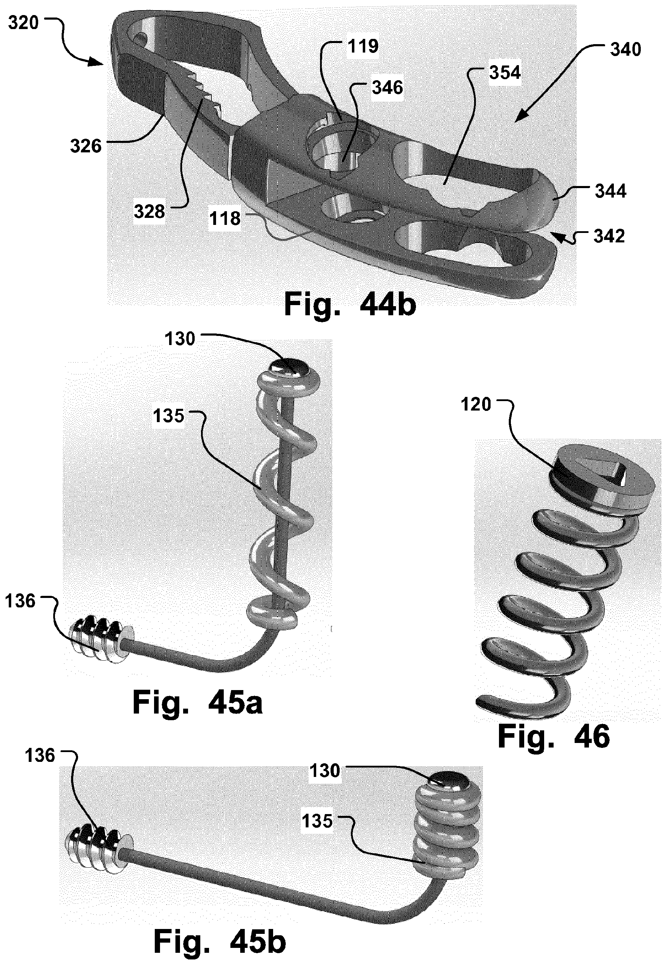

A spring attachment hole 348 is provided for spring 135 and receiving wire 130 therethrough. Wire 130 is arranged into the adjacent chain element. The wire end 136, which may include an attachment element such as a threaded element as shown in the Figs. 45a-b, is provided to engage with hole 325 of the adjacent chain segment so that drawing the wire/drawstring 130 will move the two chain segments towards each other (foreshortening). The wire 130 (and spring 135) is in examples arranged detachable for removal after foreshortening and locking of the chain ring segments. This may advantageously be implemented by unwinding a threaded wire end 136 or other removable detachable unit(s). The wire 130 (and spring 135) can then be removed from the body.

Other examples of chain segments only comprise a first end portion 320, typically the end pieces of the chain.

Some examples of chain segments have two first end portions or two second end portions connected via a center piece, like a middle piece for a symmetrically built chain ring. A chain ring is in some examples built symmetrically, including "mirrored" chain elements around such center piece chain segment.

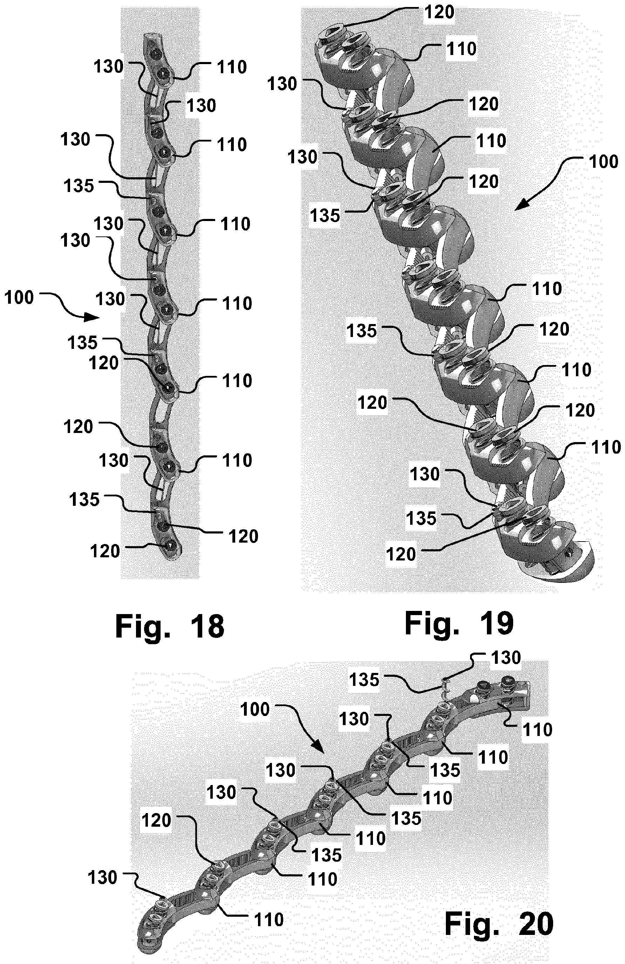

The tissue anchor 120 is arranged in a movable state at least partially within the chain segment 110 in the delivery configuration, see e.g. Figs. 18-22. In this manner, a delivery catheter 155 with a minimum inner diameter can be used for delivery as no tissue anchor 120 protrudes radially from the annuloplasty implant during delivery until deployment.

The tissue anchor 120 is suitably moved to protrude from the chain segment 110 in the deployment configuration in order to anchor with adjacent cardiac tissue into which the annuloplasty implant is desired to be anchored. The tissue anchor 120 is still attached to the annuloplasty implant when made to protrude in the deployment configuration so that the annuloplasty implant is securely anchored to the tissue. Transition from the recessed position within the annuloplasty implant to the protruded position may be provided by suitable ways, e.g. a threaded advancement, a spring-loaded release, a swellable member pushing the anchor outwardly, etc. An example of threaded advancement is described further below.

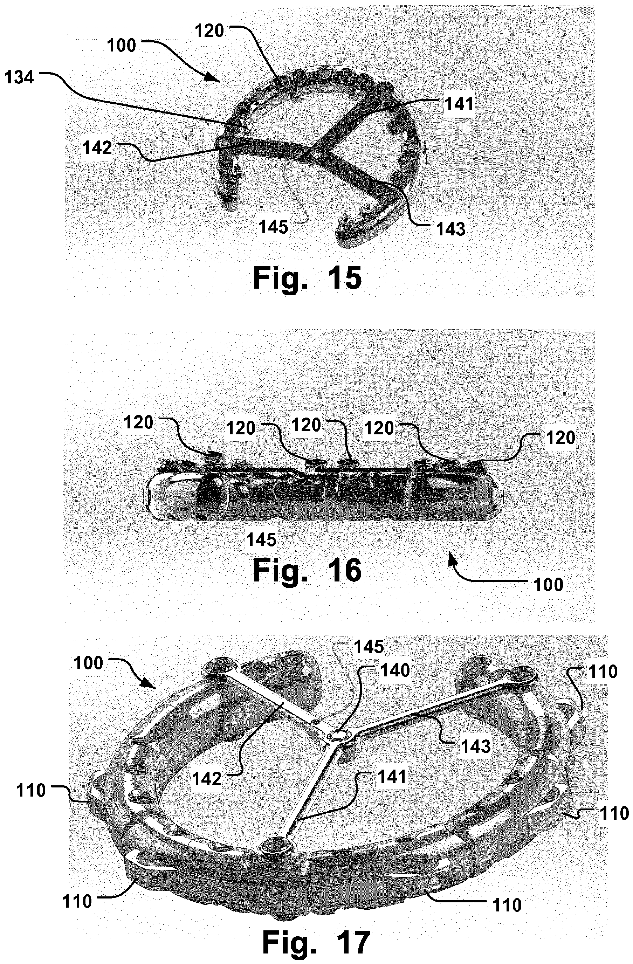

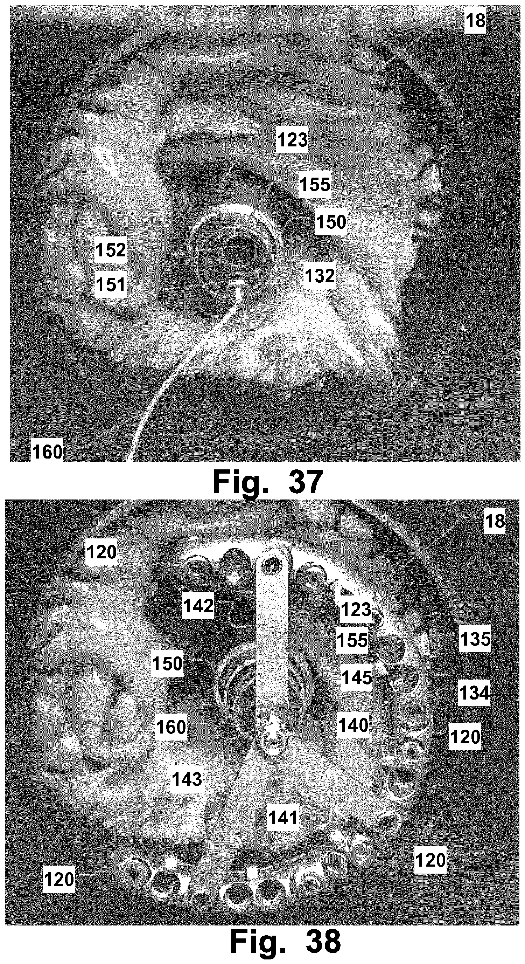

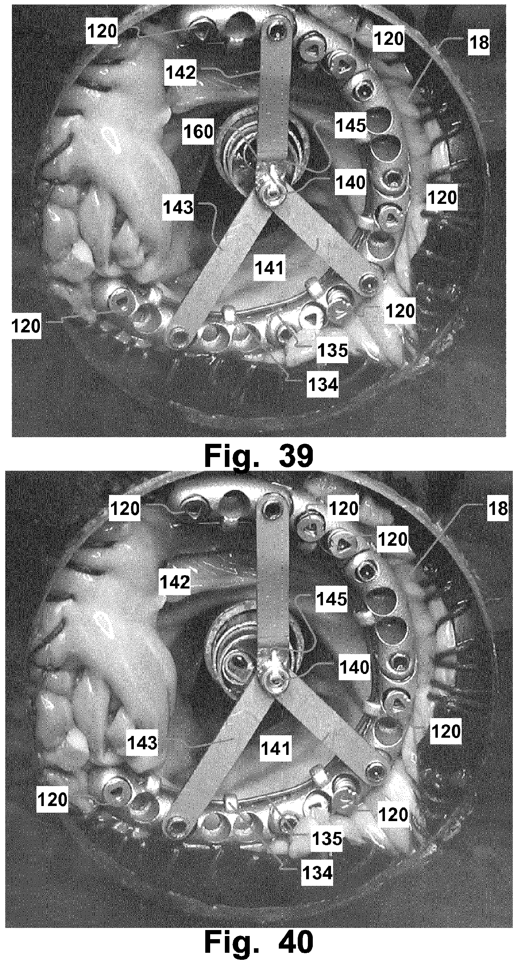

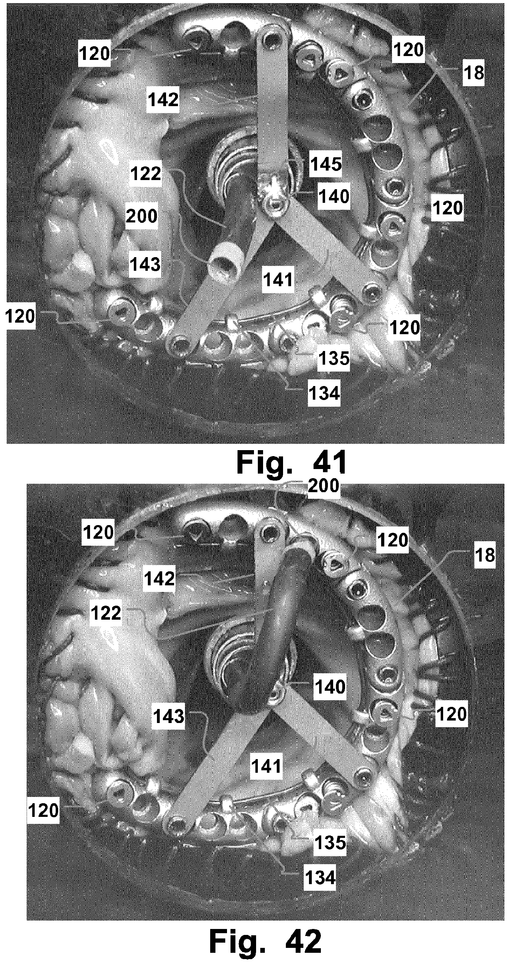

In examples the annuloplasty ring 100 can be of an open curved annuloplasty shape when deployed. Examples is a C-shaped annuloplasty ring configuration when deployed. The two opposite end segments 110 of the elongate chain constitute the ends of the open curved annuloplasty ring 100 in this case. Examples are shown in Figs. 3, 4, 5, 15, 16, 17, 25, 26 and 38 to 42.

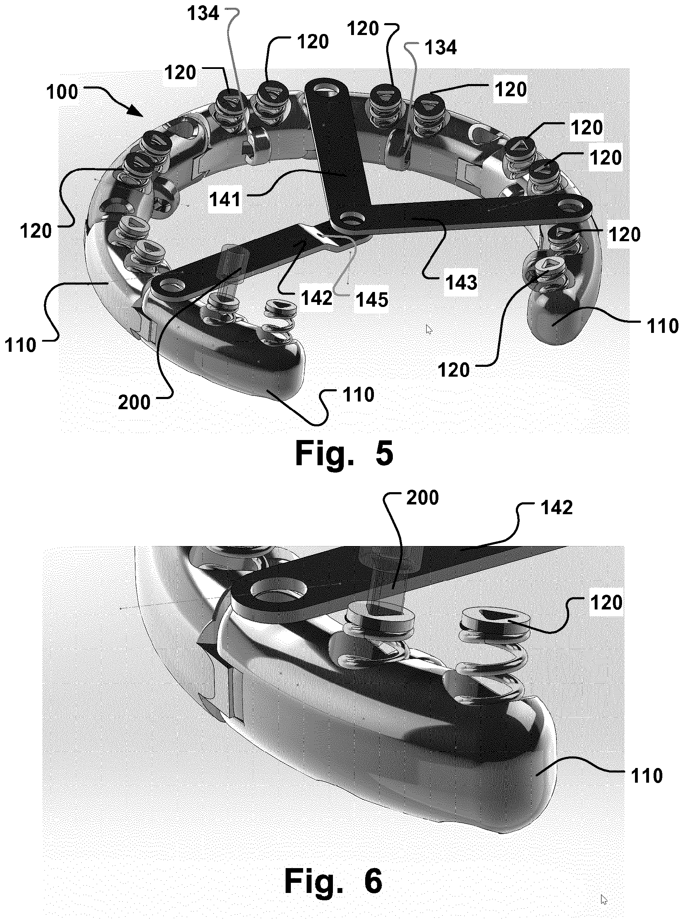

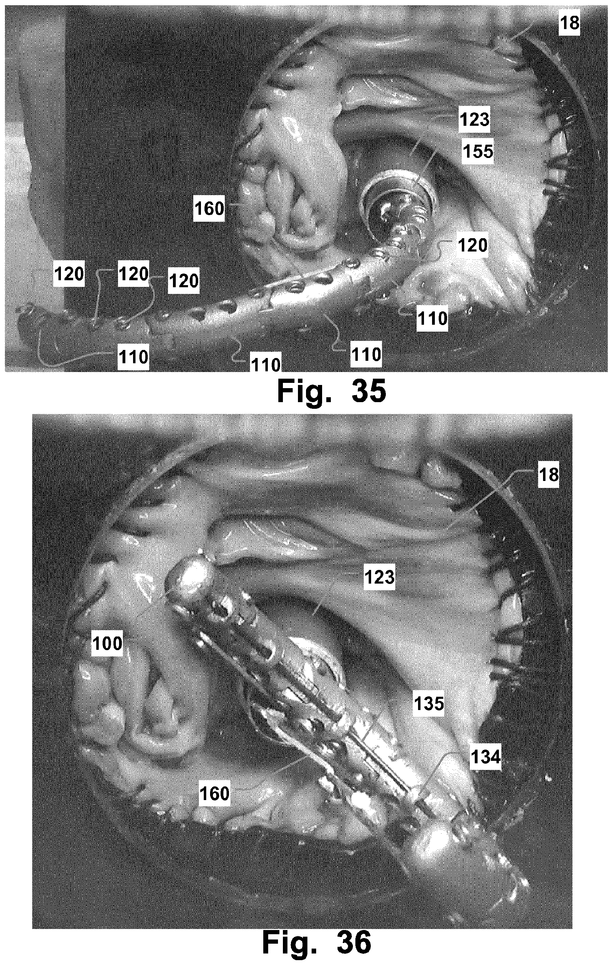

Specific chain end segments 110 may be provided, see e.g. Fig. 5 and 6, 15, 16, 17, 22, 36 and 38-42. The end segments may be rounded to be tissue friendly and allow good endothelialisation when the ring is embedding into the cardiac valve tissue upon implantation.

Alternatively the annuloplasty ring 100 can be of a closed loop shape (not shown), such as a circular, oval, D-shape, etc. The two opposite end segments 110 of the elongate chain are suitably brought together and connected to each other upon deployment of the chain in order to close the loop. The two end segments 110 can be attached to each other by suitable fastening units. Alternatively, or in addition, the two end segments may be magnetic with opposite polarities at the mating ends for a magnetic closure of the chain in the body at the cardiac valve. A chain link element may be attached between the two end segments to close the chain loop. Alternatively, or in addition the closure may be provided by inserting an element connecting the first and last chain elements of the chain together after deployment. The annuloplasty ring 100 is a flexible ring, at least in the delivery configuration and upon deployment. The ring can be stiffened up or locked to a non-flexible configuration after deployment, e.g. by stabilizing arms, such as arms 141, 142, 143 shown in the Figures.

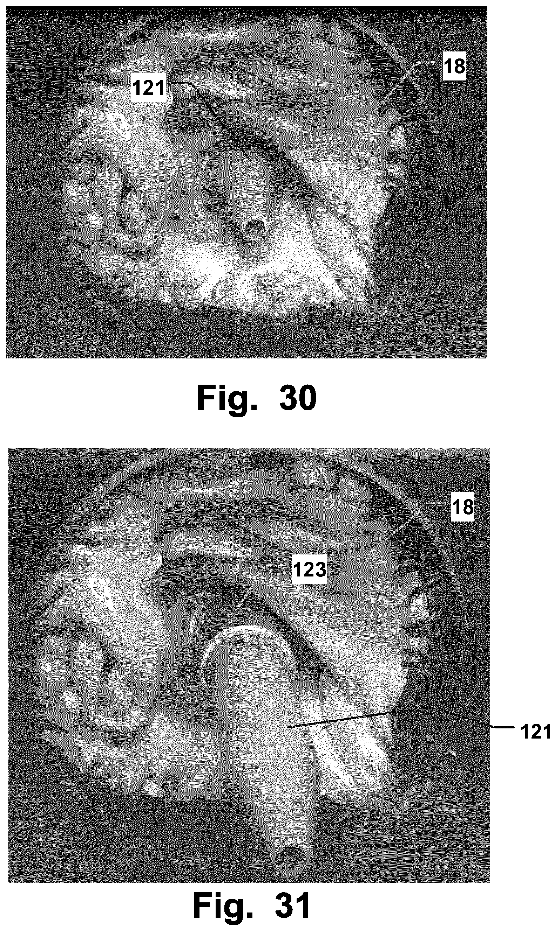

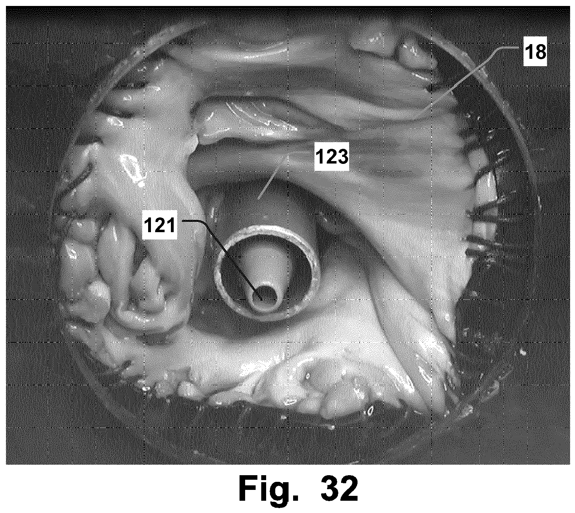

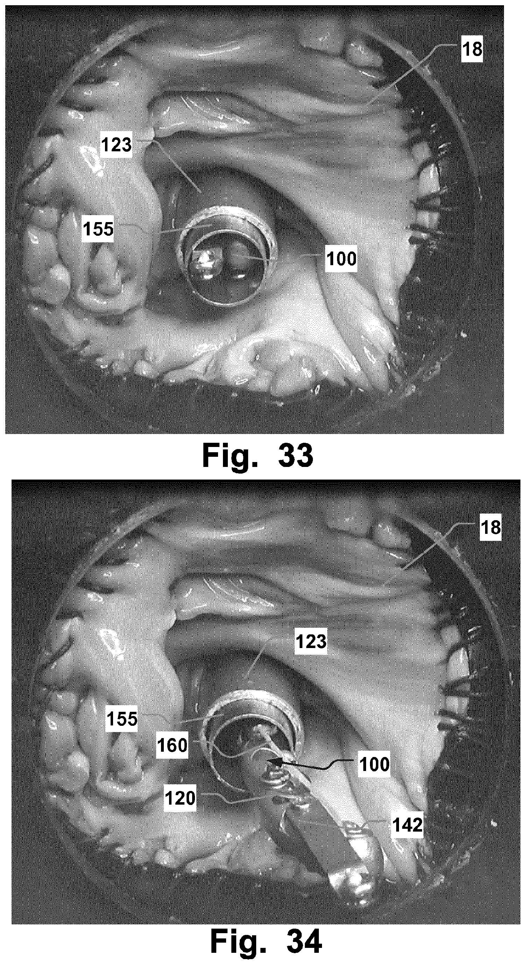

One or more arms 141, 142, 143 may serve several advantageous purposes. Firstly, during delivery of the implant they serve as a holder for being positioned into the correct cardiac tissue location from the catheter. The implant is easily graspable by means of the arms, see e.g. the example of delivery illustrated in the Figures 30 to 42. Secondly, when locked in place and the delivery system is withdrawn, they serve as stabilizing units for the chain ring 100. The one or more arms may however be removed after anchoring of the chain ring, e.g. for a chain ring that does have an annuloplasty option. This may for instance be an anchor chain ring with foreshortening features. The arm(s) may be removed together with the delivery system (and thus be part of that system).

An anchor chain ring with arms may be provided without foreshortening features and serves as an anchor for a cardiac assist device for controllably assist movement of a cardiac valve plan.

An arm 142 includes in an example an eyelet 145 close to its distal end for receiving a securing tether 160. This allows for simple and secure delivery of a chain implant at reduced radiation dosage needed during delivery. The eyelet 145 can advantageously be arranged in a surface angled in relation to the longitudinal extension of the arm 142. The latter arrangement of the eyelet 145 reduces a risk of rupture when the tether 160 is tensioned.

The arms may have second (distal) ends matingly engaging each other. An example of three arms is for instance shown in the Figures. The second end of one or more arms may include a level compensation member so that a locking unit is insertable, e.g. to be positioned in the common through hole for locking the arms in the center of the chain ring, see e.g. Fig. 5.

The annuloplasty ring 100 is configured to be permanently anchored at an annulus of a cardiac valve of a patient. Preferably, one or more tissue anchors 120 are provided for this purpose. Tissue anchors 120 provide for reliable anchoring in cardiac tissue, such as preferably annulus tissue of a cardiac valve, while being directly or indirectly attached to the annuloplasty implant. In this manner, the annuloplasty implant can be secured to the cardiac tissue by means of the tissue anchor(s) 120.

In an example, a tissue anchor 120 is preferably being connected to the chain segment 110. The tissue anchor 120 has a distal tissue anchoring portion. The distal tissue anchoring portion includes in examples a helical spiral or threaded tissue anchor 120 portion for screwing the distal anchoring portion into the tissue for anchoring therein.

The proximal end of the tissue anchor 120 is configured to remain attached to the annuloplasty implant, and in more detail to a chain segment 110 of the annuloplasty implant. The proximal attachment may include a threaded segment matingly secured in a chain segment 110. Upon rotation of the thread, e.g. via screw head engagement of a suitable rotational screwing tool 200, the tissue anchor 120 advances distally towards and into the adjacent cardiac tissue (if present).

The direction of movement of the tissue anchor 120 is radially outwards from the deployed chain 100 towards the tissue. The direction may be substantially perpendicular to the longitudinal (curved upon deployment) extension of the chain 100. If desired, and as appropriate for optimal tissue anchoring, the direction of movement out of the chain element 110 may be slightly inclined in relation to the longitudinal direction. Different tissue anchoring 120 elements along the chain 100 can be provided at different inclination angles to improve anchoring of the annuloplasty implant to the cardiac tissue.

The tissue anchor 120 is in examples compressible to a compressed configuration. For example, the tissue anchor 120 has a bayonet lock arrangement at its top end as a head. The tissue anchor 120 has a helical spring section extending from the head section. By means of the head being configured to interlock with a corresponding bayonet lock seat 119 in the chain ring body, the helical spring section can be locked in a compressed state in the ring body between bayonet lock seat 119 and an opposite counter seat 118 for receiving the lower end / tip section of the helical spring section. The tissue anchor is contained in the chain element body in the compressed state until delivery/release, e.g. by screwing out of the body with screwing tool 200. In this manner, the tissue anchor can be extended to a length longer than the compressed length upon release/protrusion from the chain segment body into adjacent cardiac tissue. See for instance Figs. 43b and 44b for reference to the seats 118, 119 (compressed screw not shown).

In examples, the annuloplasty ring 100 has the shape of a ring. The ring shape may be open or closed. The annuloplasty ring 100 has an unlocked state for delivery through a delivery catheter 155 and deployment at a cardiac valve target site. The ring 100 is flexible so it can be changed from a substantially elongate delivery shape to a curved or bent ring shape at the cardiac valve tissue. Flexibility is at least partly provided by the chain segment 110 being interlinked, i.e. joined together and articulable relative each other like a chain. Flexibility may also in addition or alternatively be provided by non-preferred flexible elements or segments integrated into the chain, e.g. flexible chain segments. The unlocked state is obtained by the chain segments 110 being articulated to each other by joints segments. The annuloplasty ring 100 is preferably rigid in a direction perpendicular to the longitudinal direction of the chain 100 curvature. This does not necessarily mean the ring 100 is flat with a curvature substantially in a single plane (which is preferred), it may in examples have a three dimensional shape like a saddle shape or other topographic shape adapted to a 3D anatomical curvature of a cardiac tissue, e.g. an annulus of a cardiac valve. The transverse or perpendicular rigidity is for instance obtained by one or more of the joints being rotatable in one plane around a joint axis only respectively.

The chain length of the annuloplasty ring 100 is for instance adjustable by at least two adjacent chain segments 110 being movably arranged relative each other. The adjacent segments 110 are preferably arranged to foreshorten relative to each other upon deployment, and more preferably after anchoring to cardiac tissue by at least the tissue anchors 120 attached to the chain 100 made protruding and become anchored to the cardiac tissue. The foreshortening of the chain 100 thus provides for the annuloplasty, i.e. reshaping of annulus tissue to treat regurgitation of the cardiac valve.

Foreshortening may be provided by a drawstring, wire 130 or other tether for pulling at least two adjacent chain segments 110 towards each other. Pulling on the string 130 will move the two chain segments 110 relative towards each other reducing the total length of two adjacent chain segments 110 respectively. The reduced length can then be locked by suitable locking units 370, which are preferably configured to lock the length non-reversible.

At least one of the chain segments 110 is bent in a longitudinal direction. The bent segment is arranged to be foreshortened relative to an adjacent linked chain segment 110 so that a curvature at the linked segments 110 is adjustable when the length of two linked chain segments 110 is shortened. The curvature of the chain 100 is thus changed when two adjacent chain segments 110 are moved relative each other. For instance by foreshortening the total length of two adjacent chain segments 110, e.g. by sliding the one chain segment 110 over or into the other chain segment 110, not only the length is made smaller, but also the radius of the curve described by the two chain segments 110. This change of curvature upon change of total chain segment length in relation to chain segment 110 curvature is illustrated more detailed herein and with relation to the Figures.

The annuloplasty ring 100 includes more than two preferably bent chain segments 110 which are arranged to foreshorten individually relative of the adjacent chain segments 110 respectively. Some adjacent chain segment 110 may not be foreshortenable relative each other, depending on the desired curvature to be obtained after potential foreshortening of the segments 110. In this manner the curvature of the ring 100 is adjustable differently at different interlinked chain segments 110. A desired total curvature of the chain ring 100 is determinable by its chain segments 110. Variable adjustability of chain segments 110 in relation to each other upon delivery allow for leeway in the annuloplasty actually performed. This is particular advantageous as a single chain ring 100 can be used for many different patients and therapies. Adjustment by foreshortening individual or all chain segment 110 is preferably based on imaging based feedback, like Doppler ultrasound regurgitation determination, contrast media based X-ray blood flow determination, MR imaging etc.

Chain elements 110 may be provided with different fixed curvature from each other. Alternatively or in addition, the total length of chain segments 110 may be different in some examples. Alternatively, or in addition, different pairs of chain segments 110 of the chain ring 100 may have different adjustable total length and thus curvature of two adjacent chain segments 110. In this manner, flexibility regarding different patient's anatomical prerequisites and therapeutic needs can be taken in consideration when choosing a specific chain ring 100 before implantation. The chain ring's curvature and thus shape are therefore flexible and adaptable, even for specific adjustments by the surgeon after deployment of the chain ring 100 at the cardiac tissue site. An example is that the same chain ring 100 may be brought from the delivery configuration to a C-shape, or a horseshoe shape, which is a more elongate C-shape.

Preferably the chain ring 100 has a final, fixated, shape upon delivery and anchoring, as well as the shape change for the annuloplasty that is an at least partly circular shape, partly oval shape, horseshoe like shape, C-shape, or similar shape.

The final shape may be pre-determined based on specific patient treatment needs. Input data from imaging modalities may be provided for simulating a desired shape of e.g. a cardiac valve annulus, desired coaptation of valve leaflets, etc. for improved valve function after treatment, e.g. reduced regurgitation. The structure of the chain ring 100 annuloplasty can thus be produced based on this desired treatment result.

Length, sizes, curvature, number of chain segments, etc. can be determined from this desired result final shape. Further input will be available mode and path of delivery, including available minimum and maximum diameter and curvature of access paths, inner and outer diameter of delivery catheters, bendability of catheters, etc.

The final shape of the chain ring 100 after deployment may be based on an average population and desired treatment result.

The adjacent chain segments 110 in the annuloplasty ring 100 include matingly arranged locking element 370 for non-reversible foreshortening of the total length of the two adjacent chain segments, as for instance a snap-in locking element.

The tissue anchor 120 is in some examples arranged as a stop element to prevent foreshortening of two adjacent chain segments 110 when arranged at least partly within said chain segment 110 prior to being arranged to protrude from said chain segment 110.

In specific examples, the tissue anchor 120 is arranged as a removable stop element in the delivery configuration. The stop element 120 prevents a foreshortening of two adjacent chain segments 110. When the stop is removed, the two chain elements 110 can be foreshortened, which may be done non-reversible.

In this manner, the tissue anchor 120 prevents as a removable stop element that an annuloplasty can be done unintentionally upon delivery. Such a stop element is in particular advantageous when the foreshortening is non-reversible and should not occur unintentionally or accidently.

The tissue anchor 120 is in an example a stop screw 120 that prevents non-reversible foreshortening e.g. before the annuloplasty ring 100 implant is anchored in the cardiac valve tissue. The tissue anchor 120 thus prevents an annuloplasty to be done unintentionally upon delivery and improves delivery security and safety of procedure and patient safety.

In particular, the tissue anchor's 120 proximal portion may be provided as a screw, i.e. with a proximal part that is threaded and matingly engages a threaded bore in the chain segment 110. The distal end of the tissue anchor 120 screw is shaped for secure anchoring in tissue, for instance cork screw shaped for secure anchoring in the cardiac tissue, like the annulus tissue.

The curved deployment configuration of the annuloplasty chain ring 100 is for instance provided by inwardly actuating spring elements 135 between adjacent chain segments 110. In the substantially straightened out elongate delivery configuration of the chain ring 100, the spring elements 135 of adjacent chain segments 110 are stretched from their relaxed configuration by straightening the chain. So that the chain ring 100 is configured to flex back from the delivery configuration to the curved deployment configuration. The initial shape after deployment of the chain ring 100 is then a curved shape with substantially relaxed spring elements 135. In case some of the chain elements are hindered to return to the springs relaxed configuration, e.g. by an anatomical or artificial structure in the body hindering a complete return, this may provide for an advantageous clamping effect preliminary fixating the chain ring 100 in position before anchoring of tissue anchor 120 in cardiac tissue and before a shape change of the chain ring 100 for an annuloplasty. Thus the chain ring 100 has a shape of an initial, first curvature upon deployment from a restraining delivery catheter 155.

The spring element 135 may be provided in an example as a tab (not shown in the Figures). The spring element 135 may also be a pop-up spring, a pre-bent elongate unit, e.g. attached with a spring element holder 134 along chain segments 110, like a pre-bent wire (see e.g. Fig. 5, 36, 38-42), for instance a pre-bent metallic wire such as made of superelastic Nitinol.

Alternatively, or in addition, the spring element 135 may be provided of a shape memory material that is activated by body temperature. In this manner, delivery through a catheter may be easier without a chain ring 100 urged into a catheter and striving back to the curved configuration. Thanks to the shape memory material used, a separate sheath around the chain ring 100 can be dispensed with, and/or a simpler delivery catheter 155 without inner glide improved lining may be used for delivery.

In an example, the ring 100 is adjustable upon deployment from the initial, first curved deployment configuration, ready for the annuloplasty, to a second curved shape of e.g. a smaller radius, for the annuloplasty. Subsequently to a change of shape to the initial first curved deployment configuration and an apposition to cardiac tissue, the tissue anchor(s) 120 are released or actively brought into tissue engagement for anchoring the chain ring 100 to cardiac tissue, preferably at an annulus such as the mitral valve annulus. The annuloplasty is then performed by changing the chain ring 100 shape to the second curved shape, thus moving cardiac tissue and remodelling the cardiac tissue. In particular if the tissue anchor 120 is a stop screw as described above, and the stop screw is moved to protrude into tissue from a chain ring segment 110, the annuloplasty can only be performed in the right sequence, i.e. after anchoring the tissue anchors 120.

In an example, the chain ring 100 includes at least one lockable arm 141, 142, 143 for stabilizing the annuloplasty chain ring to cardiac tissue at an annulus of a cardiac valve.

Lockable arms 141, 142, 143 are described in concurrently filed patent application of the same applicant with the title "An implantable cardiac valve improvement device, system and procedure" with patent application number EP18182804. This patent application is incorporated herein by reference in its entirety for all purposes. In particular the disclosure of lockable arms 141, 142, 143 for various purposes is incorporated, in particular for stabilization purposes, or connection of further elements, to the presently herein described examples of a chain annuloplasty ring 100. Fig. 5 is a 3D view of a ring similar to that of Fig. 3 with stabilizing arms attached to it.

For instance, the annuloplasty ring 100 includes alternatively or in addition at least one such lockable arm 141 that has a first end portion pivotably attached at a first circumferential position of the chain ring 100 at a chain element 110 thereof. The lockable arm 141 is preferably being pivotable in a plane stretched up by the chain ring 100 and into an inner center direction of the chain ring 100.

The lockable arm 141 can thus extend along the longitudinal direction of the chain ring 100 during delivery in the delivery configuration and be pivoted from the chain ring 100 towards the center of the latter upon deployment.

The lockable arm 141 has an opposite second end portion including a locking unit 140. At least a part of a shape of the chain ring 100 is fixed when the arm 141 is locked by the locking unit 140 relative a second circumferential position of the ring 100.

In an example the ring 100 includes a plurality of lockable arms 141 which are locked to each other at the second end portions respectively. Preferably, the arms 141 second end portions meet in the center area of the chain ring 100. An example of three lockable arms 141,142,143 is illustrated having a star configuration (e.g. approx. 120 degrees between each arm) in the Figures.

The arm(s) 141,142, 143 are locked upon finalized annuloplasty, i.e. the chain ring 100 having the second curved shape after the annuloplasty is performed by changing the chain ring 100 shape to the second curved shape as described above.

Alternatively, or in addition, the chain ring 100 is connected to a leaflet tissue via such an arm 141. The second end of the arm 141 is lockable to leaflet tissue. Locking can be done by various fastening units 140, like cardiac valve tissue locking units, such as a tissue screw anchor at the second end, a clamp, a suture, a staple, an attachment unit attachable to a patch at the leaflet surgically attached earlier to the leaflet, etc. In this manner, movement of the leaflet tissue is limited.

Suitable arms 141 are described in concurrently filed patent application of the same applicant with the title "An implantable cardiac valve improvement device, system and procedure" with patent application number EP18182804. This patent application is incorporated herein by reference in its entirety for all purposes. In particular the disclosure of arms 141,142,143 for connecting annuloplasty rings in combination with a "leaflet clip" is incorporated herein, in particular for synergistic treatment of valve regurgitation, preferably in addition to stabilization purposes, when connected to the presently herein described examples of a chain annuloplasty ring 100.

Alternatively, or in addition, the ring 100 is connected to a cardiac assist device to support movement of the cardiac valve.

Cardiac assist devices are for instance disclosed in international patent applications of the same inventor as the present application with publications numbers WO 2011/119101 A1 or WO 2011/119100 A1, which both are incorporated herein in their entirety for all purposes. The presently described chain ring 100 is in examples attached to the mitral valve plane and its movement is assisted during the cardiac cycle, preferably substantially along a cardiac long axis. Other cardiac valves may thus likewise be assisted for improving cardiac function, e.g. the tricuspid valve.

Suitable arms 141,142,143 for connection to a cardiac assist device are described in concurrently filed patent application of the same applicant with the title "An implantable cardiac valve improvement device, system and procedure" with patent application number EP18182804. This patent application is incorporated herein by reference in its entirety for all purposes. In particular the disclosure of arms for connecting annuloplasty rings to cardiac assist movement generating units is incorporated herein, in particular for synergistic treatment of valve regurgitation, preferably in addition to stabilization purposes, and/or preferably in addition to the afore described "clipping" of valve tissue, when connected to the presently herein described examples of a chain annuloplasty ring 100.

Alternatively, or in addition, the ring 100 is connected to a cardiac valve replacement or repair unit via a coupling unit like arms 141, 142, 143. A system is thus provided that includes an implantable medical device including an anchor unit (herein an example of a chain annuloplasty ring) configured to be permanently anchored at a cardiac valve of a patient. The system includes at least one coupling unit for connecting the anchor unit to a further unit, such as a cardiac valve replacement or repair unit, preferably via a locking unit 140 affixable to said further unit. The anchor unit is thus connected to the further unit, such as a cardiac valve replacement or repair unit, preferably being connected to each other via said at least one coupling unit. Suitable arms 141, 142, 143 are described in concurrently filed patent application of the same applicant with the title "An implantable cardiac valve improvement device, system and procedure" with patent application number EP18182804. This patent application is incorporated herein by reference in its entirety for all purposes. In particular the disclosure of arms 141,142,143 for connecting annuloplasty rings in combination with a cardiac valve replacement or repair unit via a coupling unit is incorporated herein, in particular for synergistic treatment of valve regurgitation, preferably in addition to stabilization purposes, when connected to the presently herein described examples of a chain annuloplasty ring 100.

In an example, the ring 100 has a closed ring shape when the opposite ends of the chain are brought together after deployment

The annuloplasty chain ring 100 can in some examples be of a closed loop shape, such as a circular, oval, D-shape, etc. The two opposite end segments of the elongate chain 100 are suitably brought together and connected to each other upon deployment of the chain 100 in order to close the loop. The two end segments can be attached to each other by suitable fastening units. Alternatively, or in addition, the two end segments may be magnetic with opposite polarities at the mating ends for a magnetic closure of the chain 100 in the body at the cardiac valve. A chain link element may be attached between the two end segments to close the chain 100 loop. Alternatively, or in addition the closure may be provided by inserting an element connecting the first and last chain elements of the ring 100 together after deployment.

In an example, a system for deployment of a chain implant 100 is provided. The system includes a multi lumen catheter 150 with a first lumen 151 arranged for receiving a holding element for said chain implant 100, and at least one screwing tool 200 in a second lumen 152 of said multi lumen catheter 150 for rotational engagement with a head of a tissue anchor 120 respectively in the chain ring 100. In an example, a method of deployment of a chain implant 100 is provided. The method includes delivering a chain implant to an annulus of a cardiac valve, anchoring of the chain implant 100 to the annulus by screwing screw anchors 120 into the annulus. In case, the chain implant is not a pure anchor implant, but an annuloplasty implant, the method includes optionally foreshortening the chain implant 100 for the annuloplasty. The method further optionally includes stabilizing of the chain implant by locking one or more arms 141, 142, 143.

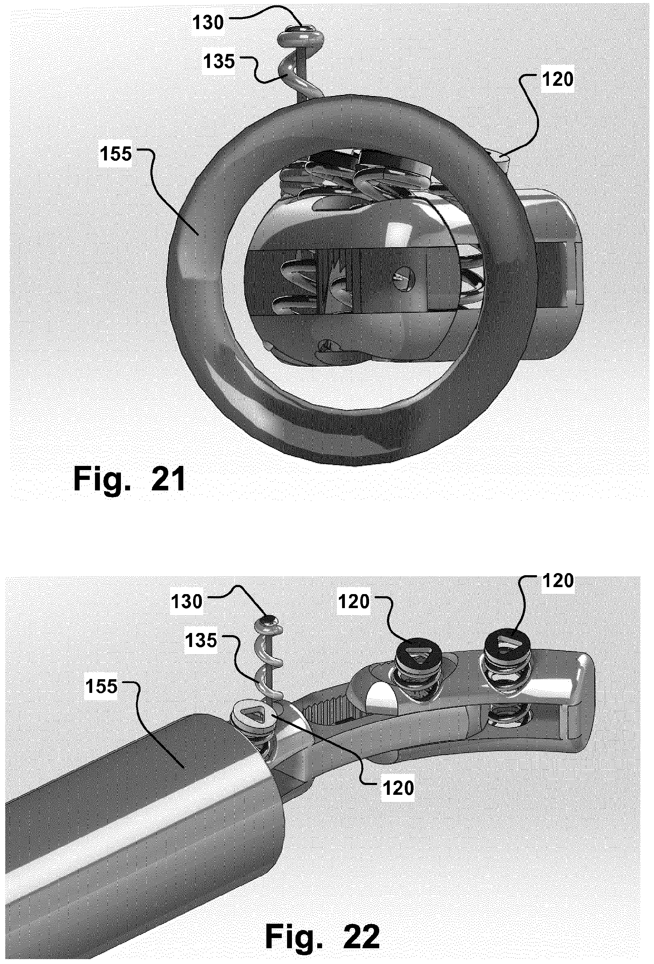

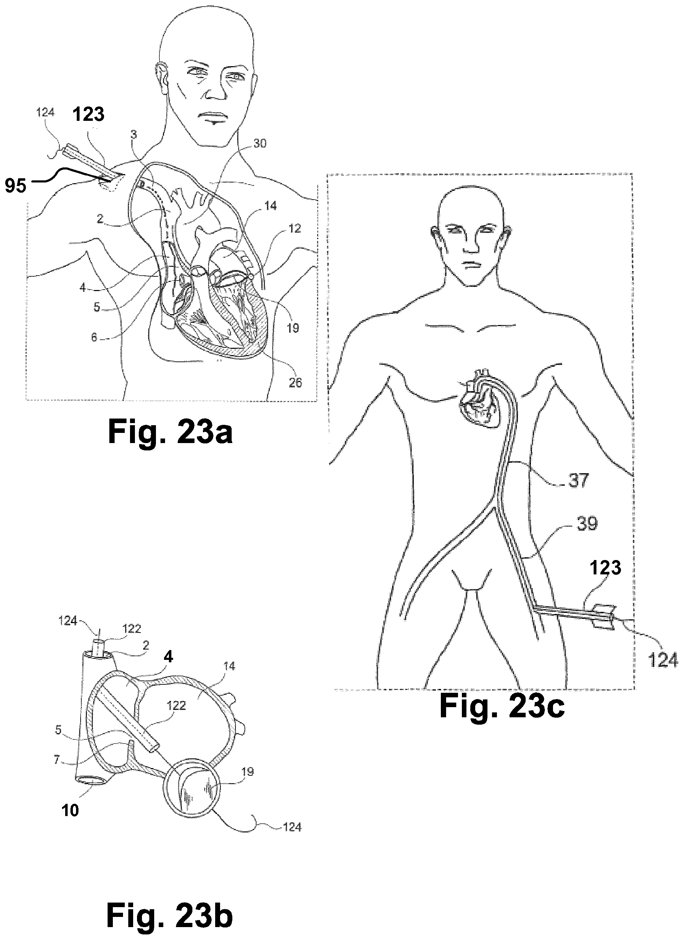

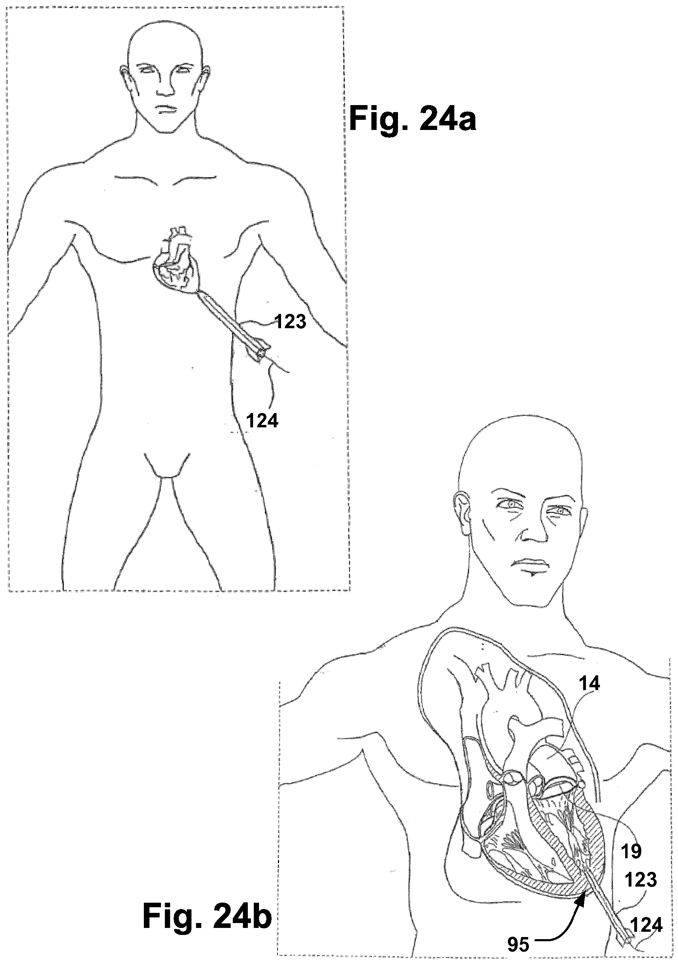

Fig. 21 is a schematic illustration of the annuloplasty ring in an inside view from the lumen in a delivery catheter 155. Fig. 22 is illustrating a delivery view of a chain annuloplasty ring with an end chain segment of a chain protruding from a delivery catheter 155. Figs. 23a-c are schematic illustrations of percutaneous transcatheter access paths to the heart. Fig. 24a-b are schematic illustrations that show a direct access path to a cardiac valve via an incision in the skin and a puncture 95 in the apex of the heart.

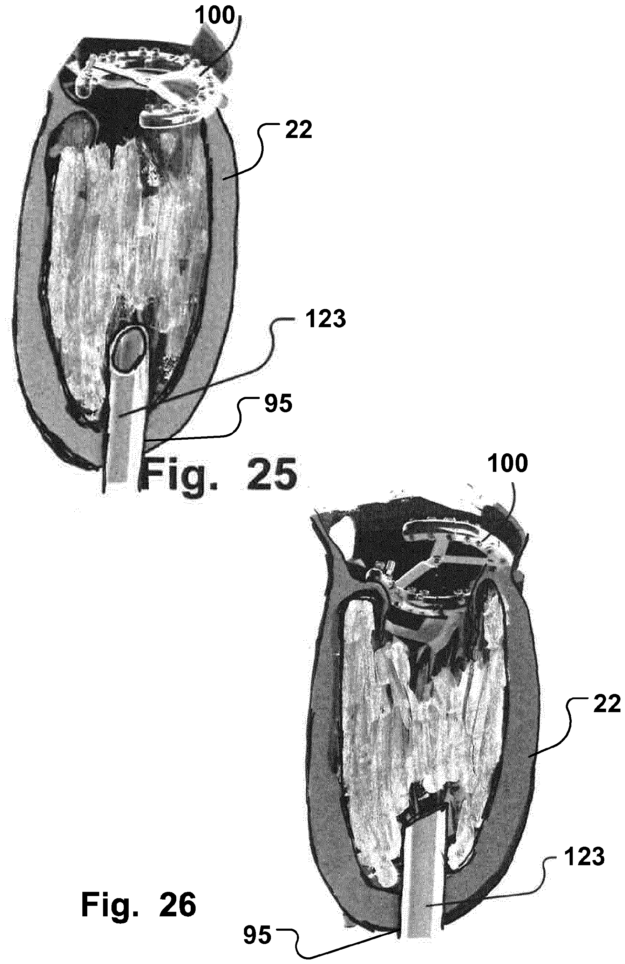

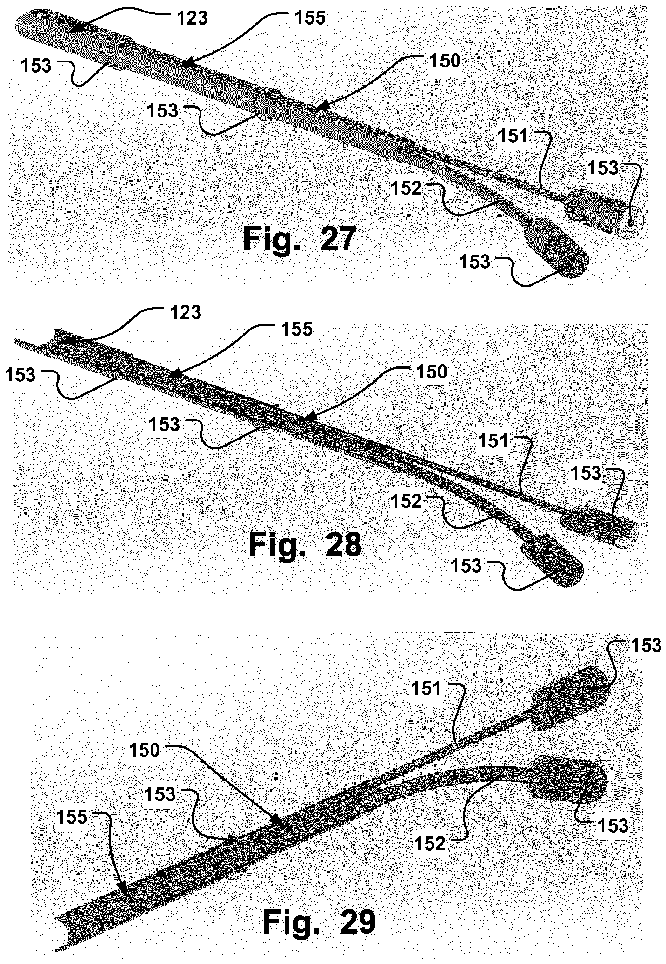

Figs. 25-26 are schematic illustrations that depict an example for complete annuloplasty ring implantation of the medical device. Fig.27 is a schematic illustration of a multi lumen catheter 150 arranged in a delivery catheter 155 and an introducer catheter 123. Figs. 28 and 29 are cross-sectional illustrations of two lumina in the multi lumen catheter 150 from Fig. 27.

In more detail, a delivery system for a medical device as described is shown with reference to Figs. 23a, 23b, 23c, 24a and 24b.

One access to cardiac valves is through the vein system as illustrated in Fig.23a. Puncture of a large vein is done at a puncture site 95. The puncture site 95 can be the neck, thorax or in the groin. An introducer catheter 123 is put in place according to common practice.

Another access to the cardiac valves is through the artery system, where an introducer catheter 123 is put in place as illustrated in Fig. 23c.

A third access to cardiac valves is through a small incision in the chest wall, giving direct access to the heart, especially the heart apex 26, again, here an introducer catheter 123 is inserted as illustrated in Fig.24a) and Fig.24b).

In common for different choices of access to cardiac valves is an armament of catheters, tubes, and wires that constitute delivery systems. A delivery system comprises a first delivery catheter 155 that may have a chain ring 100 loaded inside at the tip. Such delivery catheters 155 usually have a length that reaches from the detachment site inside of a human body to outside the body, allowing direct contact with a delivery site.

A pusher tube 132 that has a smaller outer diameter than the inner diameter of the delivery catheter 155 may be advanced axially forward inside the delivery catheter 155, in order to push the chain ring 100 out of the delivery catheter 155 at the desired site, preferably at a valve annulus.

Alternatively, the delivery catheter 155 may be retracted over the pusher tube/catheter 132, in order to deliver the device without any axial movement.

The delivery system also includes guide wires 124 that may guide the delivery catheter 155 to the intended site. The guide wire 124 may run inside the delivery catheter 155, or next to devices, or have a separate lumen in a diagnostic/guiding catheter 122.

Using similar technique, a chain ring 100 is loaded inside a delivery catheter 155 in order to be inserted into heart cavities preferably the left or right atrium of a heart. Space is accommodated inside the delivery catheter 155 for the chain ring 100 to be delivered and attached adjacent a cardiac valve.

After the chain ring 100 has been pushed out of the delivery catheter 155 by a pusher catheter 132, a multi, such as double, lumen catheter 150 may be inserted into the delivery catheter 155. Alternatively, the multi lumen catheter may already be inserted into the delivery catheter 155 and may first be used as the pusher catheter 132, i.e. no additional pusher catheter 132 is needed in this case. In the first lumen 151 of the multi lumen catheter 150, holding units may be inserted in order to position and hold the chain ring 100 in place. In a second lumen 152 of the multi lumen catheter 150, suitable tools such as screw driver 200 may be inserted with a guiding catheter 122 in order to fixate the tissue anchor 120 to the cardiac tissue.

The pusher tube 132 and/or the delivery catheter 155, accommodates a lumen for the guide wire 124 that also is permitted to run inside or next to the chain ring 100, or alternatively have a separate lumen in a diagnostic/guiding catheter 122. Chain ring 100 is released for attachment to cardiac valve tissue permanently. One or more arms 141,142, 143 may be accommodated in a delivery catheter 155.

In an example of a medical procedure of implanting a medical device as described herein is disclosed. The procedure includes implementation of the elements described above. Initially an introducer catheter 123 is placed into a chosen vessel or a heart cavity. An introducer dilator 121 may also be used in connection with the introducer catheter 123 in all descriptions below facilitating its insertion.

There are different scenarios:
In a first scenario a vein access is described as illustrated in Fig.23a, preferably a jugular vein on the neck, a subclavian vein on the thorax, femoral vein or more peripheral veins.

Once the introducer catheter 123 is in place, a diagnostic/guiding catheter 122 is inserted through the introducer catheter 123, and by means of a guide wire 124, placed adjacent to the delivery site adjacent to a cardiac valve.

Navigation inside the body is guided by means of x-ray such as fluoroscopy or CT scan and by means of ultrasound apparatus.

A guide wire 124 is left in place, allowing a delivery catheter 155 to travel over the guide wire 124 to the desired site. In case that the tricuspid valve, between the right atrium and the right ventricle is the target, the guide wire 124 is positioned in the right atrium.

If the target is the mitral valve, a trans-septal puncture of the inter atrial septum 7 is done as illustrated in Fig. 23b, and a penetration with guide wire 124 and diagnostic/guiding catheter 122 through the atrial septum between the left and the right atrium is necessary as illustrated in Fig. 23b.

Once inside the left atrium, a guide wire 124 is left inside the left atrium. Over the guide wire 124 a delivery catheter 155 may advance over the guide wire 124.

If the aortic valve is the target, a guide wire 124 and delivery catheter 155 may advance through the mitral valve into the left ventricle, facing the aortic valve from below.

In a second scenario an arterial access is preferred as illustrated in Fig. 23c., where a puncture of a large artery give access to the aorta by means of an introducer catheter 123. By means of guide wires 124 and diagnostic/guiding catheters 122, a guide wire 124 is placed above or below the aortic valve, allowing a delivery catheter 155 to access the desired delivery point.

If the mitral valve is the target, guide wire 124 and diagnostic/guiding catheter 122 may be advanced into the left ventricle from the aorta and even further from the left ventricle into the left atrium in order to get access to the mitral valve from above as well as from underneath.

In a third scenario an access from the heart apex is desired to get access to cardiac valves directly as illustrated in Fig.24a) and Fig.24b). Preferably the mitral valve and aortic valve are accessed through the left ventricle cavity, and the tricuspid valve and the pulmonary valve from the right cavity. Through a small incision in the thoracic wall and the pericardium direct access to the heart surface is obtained.

If the mitral valve is the target, an introducer catheter 123 is inserted into the left ventricle, and placed adjacent to the mitral valve, giving access to the mitral valve and its annulus from above or below.

A guide wire 124 may be used or considered unnecessary if the introducer catheter 123 is in the left atrium.

Once a guide wire 124 or a catheter is located next to the insertion site, the procedure is equal for all scenarios, therefore only the insertion of the here presented medical device will be described for the mitral valve from the left ventricle apex.

If the valve is native with no implant adjacent or in the valve, a procedure may be explained as follows:
With a guide wire 124 in place adjacent to the valve, the delivery catheter 155 is advanced over the guide wire 124 to the insertion site. A chain ring 100 is advanced through the delivery catheter 155, extruded by means of the pusher tube 132 and unfolded.

Utilizing tissue anchor units 120 the chain ring 100 is attached to the valve annulus or adjacent to it. Such chain ring 100 may by insertion have one or more coupling units like arms 141, 142, 143 attached already, preferably flexible attached to be unfolded. However, in case the coupling units are not attached to the chain ring implant 100 a coupling unit will be advanced through a delivery catheter 155 to the chain ring implant 100 and secured to it by means of an attachment unit.

The fixation by the tissue anchors 120 may be done by a suitable tool like screwdriver 200 introduced through the second lumen of the multi (double) lumen catheter 150.

Once the assembly of the new medical device is completed inside the heart, guide wires 124 and all catheters, including introducer catheter 123, are withdrawn, and the insertion site 95 is secured in order to prohibit bleedings.

Figs. 30-42 are schematic step illustrations of such a method for deployment of a chain annuloplasty implant 100.

The procedure and method described above have successfully been implemented to insert chain ring implants 100 in animals.

In another example, a method of treating a cardiac valve is provided. The method includes performing an annuloplasty by foreshortening an anchored chain ring implant and preferably patient specific adaptation of a curvature of said ring implant; optionally stabilizing said chain ring implant with one or more arms 141, 142, 143; and optionally connecting to said arms a cardiac valve tissue locking unit, a cardiac assist device, or a valve repair or replacement unit.

### Assembly of a chain ring

The assembly and mounting of the separate chain segments 110 is illustrated with reference to Figs. 43a-b, 44a-b, 45a-b, and 46.

Two chain segments 110 are put together by first sliding the left side (first end portion 320) of the chain segment 110 in Fig. 44a into the slit on the right side (second end portion 340) of the chain segment 110 in Fig.43a.

The chain segments 110 are then locked together by inserting the attachment unit 350 in Fig. 43a in between the two chain segments 110, where for instance a snap-in locking element e.g. of ratchet type as in Fig. 43a prevents the attachment unit 350 from falling out.

Once the chain segments 110 are assembled, the attachment unit 350 is included in the chain segment 110, and becomes an integrated part of the chain segment 110.

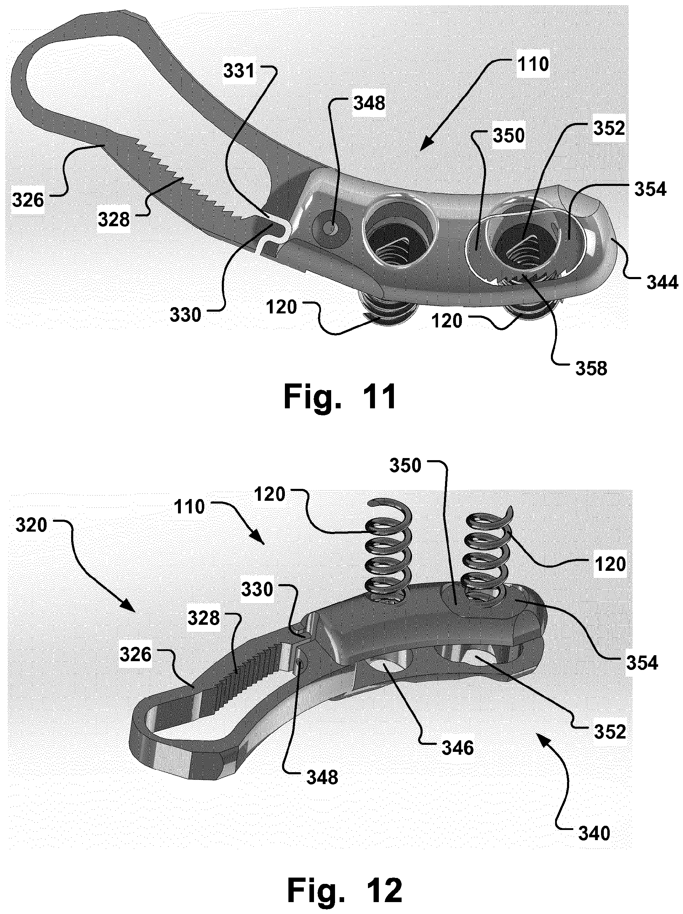

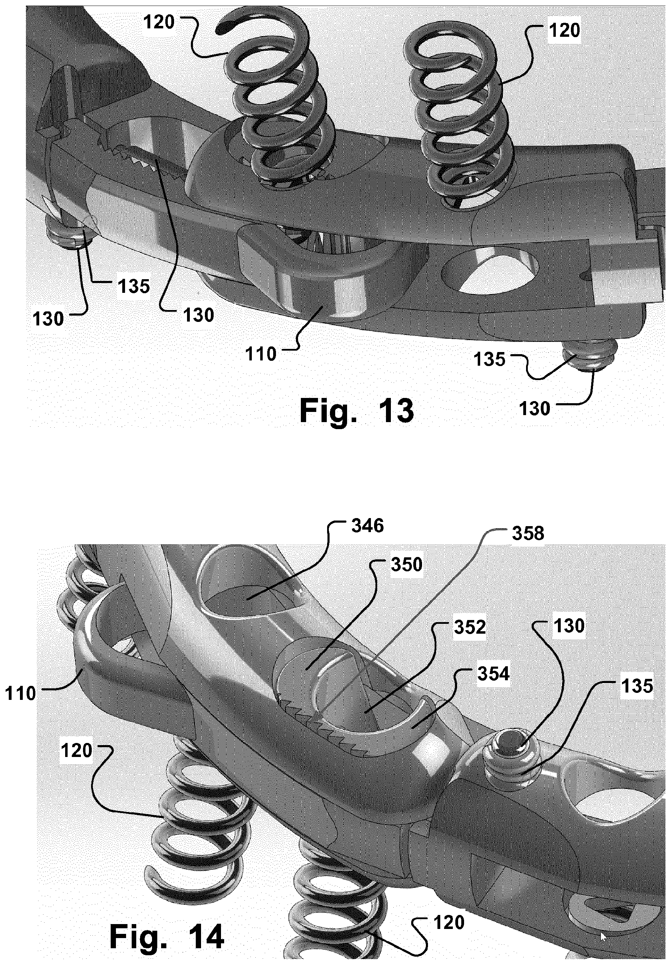

Besides from locking the chain segments 110 together, the attachment unit 350 may also be configured to include a matingly arranged locking element 358 (see e.g. Figs. 11, 14) for non-reversible foreshortening of a total length of two adjacent chain segments 110, such as a snap-in locking element e.g. of ratchet type as illustrated in Fig.43b, mating with a corresponding snap-in, teeth or ratchet 328 of the adjacent chain segment when assembled.

The attachment unit 350 may also be configured to include a tissue anchor 120 inside, such as a tissue screw as illustrated in Fig. 46.

The next step in the assembly process is to insert the tissue anchors 120 into the holes 346, 352 through the chain segments 110 and/or the attachment units 350 included in the chain segments 110, see Fig. 43a and Fig. 43b for illustration of the holes.

In some examples, the inserted tissue anchor 120 is arranged as a stop element to prevent an unwanted foreshortening of two adjacent chain segments 110. For instance, the tissue screw shown in Fig. 46 will act as a screw stop if inserted into the hole 346, i.e. on the left side of the hole 354 intended for the attachment unit 350 in Fig.43a, and thus an unwanted foreshortening of two adjacent chain segments 110 will be prevented. Once the tissue anchor is moved to protrude from the chain element 110, the stop function is removed.

The next step in the assembly process may be to insert a drawstring 130 and/or a spring element 135, for instance in the form of a spring around the proximal portion of a wire with a threaded attachment element 136 at the other end of the wire as illustrated in Fig. 45a and Fig. 45b.

The end 136 of the drawstring 130 which includes the attachment element is inserted into the small hole 348 on the top of the chain segment 110 in Fig. 43a (i.e., the small hole on the left side of the hole 346 intended for the screw stop). The attachment element 136 on the end of the drawstring 130 is then attached to the adjacent chain segment 110, for instance by screwing the threaded attachment element 136 into a threaded hole 325 on the side of either the adjacent chain segment 110 or the attachment unit 350 included in the adjacent chain segment 110. In this way, a foreshortening of two adjacent chain segments 110 may be provided by pulling on the drawstring 130 in order to move the two adjacent chain segments 110 relative towards each other for reducing the total length of two adjacent chain segments 110 respectively.

Once all the chain segments 110 are serially interlinked and articulated to a chain with all its parts assembled, the chain may be loaded into a delivery catheter 155. In order to load the chain into a delivery catheter 155, the spring elements 135 are pushed down/back, and the separate chain segments 110 are stretched such that the chain takes its elongate delivery configuration, and then the chain is pushed into a delivery catheter 155.

The two figures, Fig. 43b and Fig. 44b, are the same figures as, Fig. 43a and Fig. 44a, but shown from a different view where the chain segments have been turned 180 degrees around.

### Description of different chain configurations

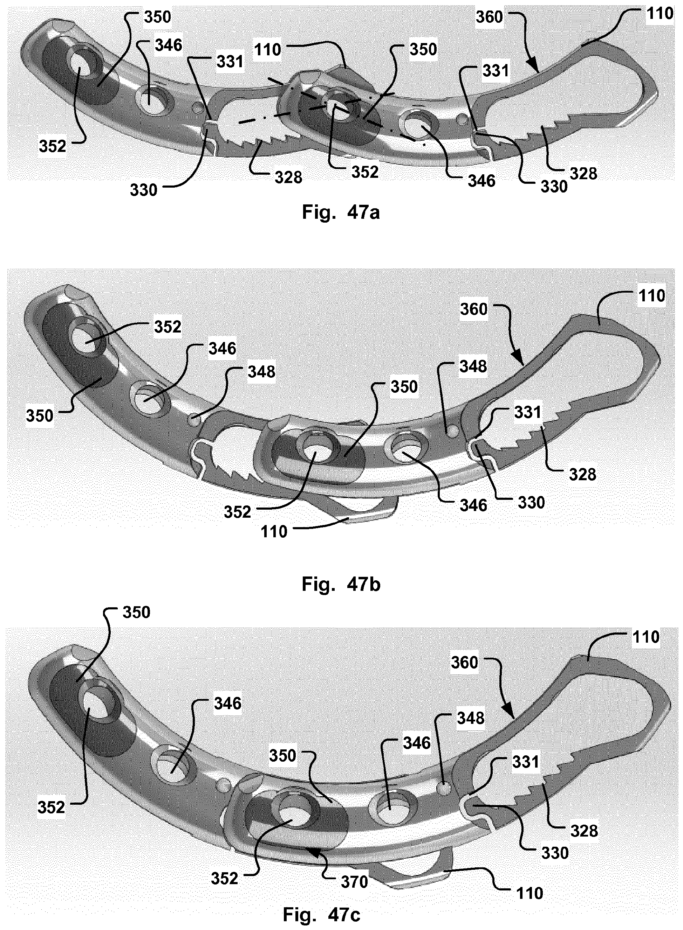

The at least partial annuloplasty ring 100, comprising a plurality of separate chain segments 110 serially interlinked and articulated to a chain, have three distinctively different configurations as illustrated in Fig. 47a, Fig. 47b, and Fig. 47c. The three different configurations are, a substantially elongate delivery configuration (Fig. 47a), a curved deployment configuration before foreshortening (Fig. 47b), and a foreshortened curved shape configuration of smaller radius than the deployment configuration, i.e., an annuloplasty configuration (Fig. 47c).

The delivery configuration is obtained by pushing down/back the spring elements 135, and then bending the separate chain segments 110 such that the chain takes its substantially elongate delivery configuration, see Fig. 47a. The interior groove (i.e. big hole), on the right side of the chain segment 110 in Fig. 47a i.e. the first end portion 320 has a widening in the hole on the right end which bends the hole in a knee 326 shaped manner. This specific knee 326 shaped feature in the design of the chain segment 110, allow the chain segments 110 to be bent and stretched in an almost straight manner, resulting in the substantially elongate delivery configuration.

The deployment configuration is obtained once the spring elements 135 springs back and pushes the separate chain segments 110 into a curved predetermined state. The tissue anchor 120 is arranged to protrude from the chain segment 110 in the deployment configuration to anchor with cardiac tissue. In one embodiment of the invention, the tissue anchor 120 is arranged as a stop element to prevent an unwanted foreshortening of two adjacent chain segments 110 when arranged at least partly within the chain segment 110 prior to being arranged to protrude from the chain segment 110. Such a stop element is obtained if a tissue anchor 120, such as a tissue screw illustrated in Fig. 46, is included within the hole 346 i.e. shown on the right side of the attachment unit 350 in Fig. 47b, and thus an unwanted foreshortening of two adjacent chain segments 110 will be prevented.

The annuloplasty configuration is obtained by pulling on the drawstring 130 in order to move the two adjacent chain segments 110 relative towards each other reducing the total length of two adjacent chain segments 110 respectively. Thus, an annuloplasty of a cardiac valve is obtained by the foreshortening of the two adjacent chain segments 110. The annuloplasty configuration has a curved shape of smaller radius then that of the curved deployment configuration, i.e., the annuloplasty configuration has not only a reduced total length but has also a more curved shape than the shape of the deployment configuration, see Fig. 47b and Fig. 47c. The foreshortening is non reversible when locking elements 370 like teeth 328/358 are engaged.

### Collar/Sock/Stocking/Pouch/Bag/Sack

The annuloplasty ring 100 may be covered with for instance a collar, a sock, a stocking, a pouch, a bag, or a sack. The cover material may for instance at least partly consist of polyethylene terephthalate (commonly abbreviated PET, PETE, or the obsolete PETP or PET-P, and may also be referred to by the brand names Terylene, Lavsan, and Dacron), polytetrafluoroethylene (commonly abbreviated PTFE, or stretched/expanded PTFE also called ePTFE, also more commonly known by the generic trademark Teflon or the registered trademark Gore-Tex), polyurethane (commonly abbreviated PUR and PU, and also commonly used in manufacture of synthetic fibers such as Spandex, Lycra, or Elastane), other polyester material, fabric material, or any other biocompatible materials. The cover material may for instance at least partly be threaded, laced, stringed, sewed, stitched, or sprayed upon the annuloplasty ring 100.

The cover material may be all away around the chain segments 110 covering the entire annuloplasty ring 100, but do not need to cover all the way around the chain segments 110, and may for instance only cover the side and/or the bottom of the annuloplasty ring 100. The top part of the tissue anchor 120, e.g. such as the tissue screw in Fig. 46, may protrude through the cover material in order to easy access the head of the screw. Alternatively, the top part of the annuloplasty ring 100 may be left uncovered, or there may be holes in the cover material over the holes containing the tissue anchors 120, in order to easy access the top part of the tissue anchor 120.

The cover materials have several particular advantages, for instance the cover material may be configured to facilitate a better ingrowth of the annuloplasty ring 100, the cover material may be configured to avoid any sharp edges of the annuloplasty ring 100, and/or the cover material may be configured to avoid that any cardiac tissue around the annuloplasty ring 100 get squeezed when the chain segments 110 are foreshortened. The cover material may also improve sealing of the chain ring against the valve tissue. It may thus alternatively or in addition prevent or reduce a possible backflow of blood.

### Stabilizing Annuloplasty Ring 100 with the Foreshortening

In some examples, the plurality of separate chain segments 110, serially interlinked and articulated to a chain 100, are flexible with respect to each other in the deployment configuration. The flexibility is configured to allow the different chain segments 110 to easily follow the annulus of the cardiac valve, in order to facilitate the anchoring of the tissue anchors 120 in the annulus.

Once the annuloplasty ring 100 is anchored in the annulus of the cardiac valve, an annuloplasty of the cardiac valve is obtained, in the illustrated examples by pulling on the drawstrings 130 in order to move adjacent chain segments 110 relative towards each other reducing the total length of adjacent chain segments 110 respectively.

The foreshortening of the adjacent chain segments 110 is configured to make the separate chain segments 110 stable with respect to each other, thus the flexibility in the deployment configuration goes away ones the annuloplasty ring 100 is adjusted into the annuloplasty configuration.

A matingly arranged locking element 370, e.g. provided by mating and engagingly arranged teeth 328/358, included in the chain segments 110 for non-reversible foreshortening, may be configured to obtain the stabilization of the annuloplasty ring 100. The locking element 370 may for instance be a snap-in locking element e.g. of ratchet type as illustrated in Fig. 43a and Fig. 43b. The matingly arranged locking element 370, included in the chain segments 110 for non-reversible foreshortening, may also be configured to obtain the flexibility of the separate chain segments 110, where said locking element 370 may for instance be a tapered and/or conic shaped snap-in locking element e.g. of ratchet type as illustrated in Fig. 43a and Fig. 43b.

### Individualization

In one embodiment of the invention, the chain segments 110 is arranged to foreshorten individually relative adjacent chain segments 110 respectively such that the curvature of the annuloplasty ring 100 is adjustable differently at different interlinked chain segments 110. Thus, different adjacent chain segments 110 may be foreshortened differently, i.e. the relative movement towards each other may be different for different adjacent chain segments 110. The reduction in the total length of two adjacent chain segments 110 may be anything from nothing and up to the maximum allowed reduction. In this way, the annuloplasty ring 100 may be individualized for each patient, since the same annuloplasty ring 100 may be foreshortened in multiple ways. Thus, the same annuloplasty ring 100 may be used in different patients with different need for annuloplasty, since the annuloplasty ring 100 is configured to be adaptable and to have the ability to be tailored for each individual with respect to their annuloplasty need. The individualization and the tailoring of the foreshortening of the annuloplasty ring 100 may be done during an ongoing cardiac valve treatment, e.g. by changing the foreshortening at different places until the leakage in the cardiac valve has disappeared.

### Anchoring at Cardiac Tissue

The tissue anchor 120 may be arranged to protrude from the chain segment 110 with the help of e.g. a screwdriver or some other appropriate tool, which for instance may be inserted in the right position with a multi/double lumen catheter 150. Alternatively, each tissue anchor 120 may be configured to e.g. include a wire, a chain, a string, or a thread, which is attached to the top part of the tissue anchor 120. Each tissue anchor 120 is then arranged to protrude from its chain segment 110 by turning, twisting, and/or pushing each attached wire, chain, string, or thread. In one embodiment of the invention, the wire, chain, string, or thread is configured to automatically and/or manually be detached as well as removed from the top part of the tissue anchor 120, once the tissue anchor 120 is anchored at the cardiac tissue.

Screwing tool(s) 200 may be pre-mounted to one or more tissue anchors 120 before insertion into the delivery catheter 155. In this manner, navigation and locating each tissue anchor 120 is not needed. Each tissue anchor may thus also be individually anchored in a desired sequence for improved and speedy anchoring. After anchoring the tissue anchor(s) 120 at cardiac tissue, the pre-mounted screwing tool(s) 200 may be automatically or manually detached.

In an example a deployment apparatus is provided for such a chain implant 100 described herein. The deployment apparatus includes a multi lumen catheter 150 with a first lumen 151 arranged for receiving a holding element for said chain implant 100, and at least one screwing tool 200 in a second lumen 152 of said multi lumen catheter 150 for rotational engagement with a head of a tissue anchor 120 respectively in said chain ring 100.

### Short Surgery Time

A particular advantage with the present invention is that the surgical procedure time of the annuloplasty treatment will be short, since the tissue anchor 120 is at least partly integrated within the chain segment 110 from the beginning and the surgeon do not need to put any extra time on inserting the tissue anchors 120 into the chain segments 110.

### Open Ring

In examples, the annuloplasty ring 100 can be of an open curved shape when deployed. The annuloplasty ring 100 may have the shape of an open ring, for instance the shape may be at least partly circular, semi-circular, half circular, at least partly elliptical, semi elliptical, or half elliptical. The two opposite end chain segments 110 of the elongate chain may point towards each other, in the same direction, slightly away from each other, or anything in between. The annuloplasty ring 100 may have a symmetrical shape or an asymmetrical shape.

In an example, a method of deployment of a chain implant is provided. The method is preferably for an annuloplasty, such as a chain implant of any of the examples given herein. The method includes providing a chain implant (100), delivering said chain implant to a annulus of a cardiac valve, anchoring of the chain implant (100) to said annulus by screwing screw anchors (120) into said annulus; and optionally foreshortening said chain implant (100) for said annuloplasty, and/or optionally stabilizing of the chain (100) implant by locking one or more arms (141, 142, 143.) of fixed or adjustable length to said implant

In an example, a method of treating a cardiac valve is provided. The method includes performing an annuloplasty by foreshortening an anchored chain ring implant and preferably patient specific adaptation of a curvature of said ring implant; optionally stabilizing said chain ring implant with one or more arms (141, 142, 143); and optionally connecting to said arms a cardiac valve tissue locking unit, a cardiac assist device, or a valve repair or replacement unit.

The present invention has been described above with reference to specific embodiments. However, other embodiments than the above described are equally possible within the scope of the invention. The scope of the invention is only limited by the appended patent claims.

### List of reference signs

- 1: Structures of the heart
- 2: Superior Vena Cava (SVC)
- 3: Subclavian vein
- 4: Right atrium (RA)
- 5: Foramen ovale
- 6: Coronary Sinus (CS)
- 7: Inter atrial septum
- 8: First part of the CS
- 10: Inferior Vena Cava (IVC)
- 12: Great Cardiac Vein (GCV)
- 14: Left Atrium cavity (LA)
- 16: LA wall
- 18: Mitral Valve (MV) annulus
- 19: Whole mitral valve
- 20: Anterior leaflet of the mitral valve
- 21: The posterior leaflet of the mitral valve
- 22: Left Ventricular muscular wall
- 24: Papillary muscles connected to the chordae
- 26: Apex of the left ventricle
- 28: Aortic valve
- 30: Aorta ascendens
- 32: Inter-ventricular muscular septum
- 34: Left ventricular cavity
- 36: Right ventricular cavity
- 37: Abdominal and thoracic aorta
- 38: Right ventricular muscular wall
- 39: Iliac or femoral artery
- 40: The tricuspid valve
- 48: Cardiac valve plane
- 49: Cardiac axis
- 95: Puncture site
- 100: Annuloplasty (chain) ring 100
- 110: Chain segment
- 118: opposite counter seat
- 119: bayonet lock seat
- 120: Tissue anchor/stop screw
- 121: Introducer Dilator
- 122: Diagnostic/guiding catheter
- 123: Introducer catheter
- 124: Guide wire
- 130: Wire, drawstring
- 132: Pusher tube / catheter
- 134: Spring element holder
- 135: Spring element
- 136: Wire end (may include attachment element)
- 140: Locking unit
- 141, 142, 143: Lockable arms
- 145: Eyelet for securing tether 160
- 150: Multi, e.g. double lumen catheter
- 151: First lumen of catheter 150
- 152: Second lumen of catheter 150
- 153: Sealing valve
- 155: Delivery catheter
- 160: Securing tether
- 200: Screwing tool
- 320: First end portion of chain segment 110
- 322: 1st axis
- 324: 2nd axis
- 325: Hole for wire end 136
- 326: Knee
- 328: Teeth
- 330: Toe
- 331: Gap
- 340: Second end portion of chain segment 110
- 342: Slot
- 343: Stop
- 344: Rounded end
- 346: Second anchor hole
- 348: Spring attachment hole
- 350: Attachment unit
- 352: First anchor hole
- 354: Oval shape hole
- 358: Teeth of attachment unit
- 360: Curvature
- 370: Locking element for chain segments

## Claims

1. An at least partial annuloplasty ring (100) for transcatheter cardiac valve treatment, comprising a plurality of separate chain segments (110) serially interlinked and articulated to a chain (100), said chain (100) having a substantially elongate delivery configuration and a curved deployment configuration,
at least one of said chain segments (110) including at least one attached tissue anchor (120), said tissue anchor (120) being movably arranged at least partly within said chain segment (110) in said delivery configuration and arranged to protrude from said chain segment (110) in said deployment configuration to anchor with cardiac tissue.

2. The annuloplasty ring (100) of claim 1 , wherein said tissue anchor (120) being connected to said chain segment (110), and including a helical spiral or threaded tissue anchor.

3. The annuloplasty ring (100) of claim 1 or 2, wherein said tissue anchor (120) being lockable in a longitudinally compressed state in said chain segment (110).

4. The annuloplasty ring (100) of any of claims 1 to 3 having a ring shape with a flexible curvature in an unlocked state by said chain segments (110) being articulated to each other by joints segments and said ring (100) being rigid in transverse direction to the curvature by said each of said joints being rotatable in one plane only respectively.

5. The annuloplasty ring (100) of any of claims 1 to 4, wherein said chain (100) has a chain length that is adjustable by at least two adjacent chain segments (110) being arranged to foreshorten relative each other, and/or wherein at least one chain segment (110) is bent in longitudinal direction and is arranged to be foreshortened relative an adjacent linked chain segment (110) such that a curvature at said linked segments is adjustable when said length of said two linked chain segments is shortened.

6. The annuloplasty ring (100) of claim 5, including more than two preferably bent chain segments (110) arranged to foreshorten individually relative adjacent chain segments (110) respectively such that said curvature of said ring (100) is adjustable differently at different interlinked chain segments (110).

7. The annuloplasty ring (100) of any of claims 5 to 6, wherein adjacent chain segments (110) include a matingly arranged locking element (370) for non-reversible foreshortening of a total length of said two adjacent chain segments (110), such as a snap-in locking element (328, 358).

8. The annuloplasty ring (100) of any of claims 1 to 7, wherein said tissue anchor (120) is arranged as a stop element to prevent foreshortening of two adjacent chain segments (110) when arranged at least partly within said chain segment (110) prior to being arranged to protrude from said chain segment (110).

9. The annuloplasty ring (100) of any of claims 1 to 8, wherein said tissue anchor (120) is a stop screw in the delivery configuration that prevents a foreshortening of two adjacent chain segments (110), wherein the stop is removed upon threading the stop screw (120) into adjacent annulus tissue in the deployment configuration .

10. The annuloplasty ring (100) of any of claims 1 to 9, wherein said curved deployment configuration of said ring (100) is provided by inwardly actuating spring elements (135) between adjacent chain segments (110), such that said ring (100) is configured to flex from said delivery configuration to a said curved deployment configuration with a shape of a first radius curvature upon deployment from a restraining delivery catheter (155).

11. The annuloplasty ring (100) of any of claims 1 to 10, wherein said ring upon deployment is adjustable from a first curved deployment configuration to a second curved shape of smaller radius for said annuloplasty.

12. The annuloplasty ring (100) of any of claims 1 to 11, wherein said ring (100) including at least one lockable arm (141) for stabilizing said ring (100) at said annulus.

13. The annuloplasty ring (100) of claim 12, wherein said lockable arm (141) having a first end portion pivotably attached to said ring (100) at a first circumferential position of said ring (100) and being pivotable in a direction of a center of said ring (100), wherein said arm (141) having an opposite second end portion including a locking unit (140), such that at least a part of a shape of said ring (100) is fixed when said arm (141) is locked by said locking unit (140) relative a second circumferential position of said ring (100).

14. The annuloplasty ring (100) of any of claims 1 to 13, wherein said ring (100) including a plurality of lockable arms (141,142,143) lockable to each other at second end portions respectively.

15. The annuloplasty ring (100) of any of claims 1 to 14, wherein said ring (100) has a closed ring shape when opposite ends of said chain are brought together after deployment.

16. The annuloplasty ring (100) of any of claims 1 to 15, wherein said ring (100) is connected to leaflet tissue via an arm (141) to limit movement of said leaflet tissue, and/or said ring is connected to a cardiac assist device to support movement of said cardiac valve, and/or said ring is connected to a cardiac valve replacement or repair unit via said arm (141).

17. A medical implantable device for anchoring at cardiac valve tissue, comprising a plurality of separate chain segments (110) serially interlinked and articulated to a chain (100), said chain (100) having a substantially elongate delivery configuration and a curved deployment configuration, and at least one arm, said arm being attachable or attached, preferably pivotably attached, to one of said chain segments, and
at least one of said chain segments (110) preferably including at least one attached tissue anchor (120), said tissue anchor (120) being movably arranged at least partly within said chain segment (110) in said delivery configuration and arranged to protrude from said chain segment (110) in said deployment configuration to anchor with cardiac tissue.

18. A deployment apparatus for a chain implant (100), such as of any of claims 1 to 17, including a multi lumen catheter (150) with a first lumen (151) arranged for receiving a holding element for said chain implant (100), and at least one screwing tool (200) in a second lumen (152) of said multi lumen catheter (150) for rotational engagement with a head of a tissue anchor (120) respectively in said chain ring (100).

19. A chain segment (110) for a medical implant, including:
- at least one arm (141, 142, 143), said arm being attachable or attached, preferably pivotably attached, to said chain segment (110), said chain segment having a longitudinal extension and said arm having being arranged along said longitudinal extension in a first configuration and arranged extending radially outwards from said chain segment in a second configuration; and/or
- at least one attached tissue anchor (120), said tissue anchor (120) being movably arranged at least partly within said chain segment (110) in a first configuration and arranged to protrude from said chain segment (110) in a second configuration to anchor with cardiac tissue.
